# EUROPEAN PATENT APPLICATION

(11) **EP 4 733 766 A1**
(43) Date of publication of application: **29.04.2026**
(21) Application number: 25210617.4
(22) Date of filing: 23.10.2025
(51) Int. Cl.: G01N 33/58, C07D 237/32, C09K 11/06

(54) **LUMINESCENT LABEL, LUMINESCENT LABEL INTERMEDIATE SOLUTION AND PREPARATION METHOD THEREFOR, LUMINESCENT LABEL CONJUGATE, APPLICATIONS THEREOF, AND KIT**

(30) Priority: 25.10.2024 CN 202411504237
(71) Applicant: Shenzhen New Industries Biomedical Engineering Co., Ltd., Shenzhen, Guangdong 518122 (CN)
(72) Inventor: MA, Qiao, Shenzhen (CN); LIU, Lei, Shenzhen (CN); DU, Kai, Shenzhen (CN); WANG, Yanmei, Shenzhen (CN)
(74) Representative: Office Freylinger

(57) **Abstract**

The present disclosure provides a luminescent label, a luminescent label intermediate solution and a preparation method therefor, a luminescent label conjugate, applications thereof, and a kit. The luminescent label provided in the present disclosure has a structure as represented by general formula (I). The luminescent label provided in the present disclosure not only has good performance of luminescence intensity, but also can directly couple with a protein to generate a luminescent label intermediate under the action of an activator. The labeling process is simple, and the steric hindrance is lower during labeling, which can effectively improve the labeling efficiency while ensuring the luminescence performance.

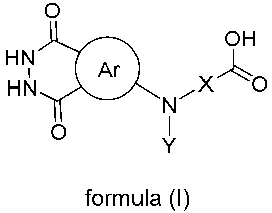

## Description

### Technical Field

The present disclosure relates to the technical field of luminescent labels, and in particular, to a luminescent label, a luminescent label intermediate solution and a preparation method therefor, a luminescent label conjugate, applications thereof, and a kit.

### Background

Chemiluminescent labels play a crucial role in in vitro diagnostics, and are widely used as highly efficient labeling tools, particularly used for labeling proteins such as antigens, antibodies, and binding proteins. This labeling technology relies on the principle of chemiluminescence, and achieves accurate quantification of the concentration of target substances by measuring the intensity of luminescent signals. Chemiluminescence immunoassay exhibits excellent sensitivity and accuracy, coupled with a simple and fast operation workflow, making it highly suitable for fast screening and deep quantitative analysis of large-scale samples. Therefore, it plays an indispensable role in various key fields, such as rapid diagnosis of infectious diseases, prediction and evaluation of the risk of genetic diseases, and analysis of target analytes in new drug research and development.

Existing luminescent labels have the problem of a conjugate labeling process in protein labeling applications, and affect the labeling efficiency of actual application processes. Therefore, there is a need for a new luminescent label, which simplifies the labeling process while ensuring the performance and improves the labeling efficiency, thereby better meeting the demands for high-efficiency, rapid and stable labeling technologies in fields such as biomedical research, clinical diagnosis and drug development.

### Summary

The main object of the present disclosure is to provide a luminescent label, a luminescent label intermediate solution and a preparation method therefor, a luminescent label conjugate, applications thereof, and a kit, so as to solve the problem in the prior art that a labeling process is conjugate and therefore the labeling efficiency of a practical application process is affected while ensuring the luminescent performance of the luminescent label.

In order to achieve the described object, according to one aspect of the present disclosure, provided is a luminescent label, and the luminescent label has a structure as represented by general formula (I): 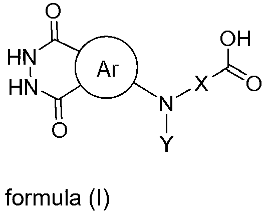
wherein Ar is any one selected from general formulas (II)-(IV): 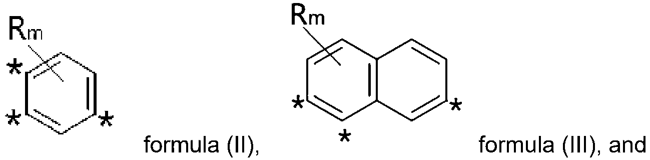 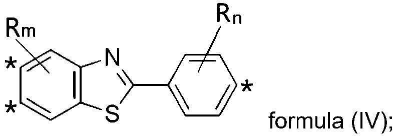
m is an integer between 0 and 2, and when m = 2, each R is the same or different; n is an integer between 0 and 4, and when n ≥ 2, each R is the same or different; R is selected from C1-C10 alkyl groups; X is selected from C1-C10 alkylene groups, and C atom or H atom thereof may be optionally substituted by O atom, S atom, an aryl group or a heteroaryl group; Y is selected from C1-C10 alkyl groups and C1-C10 alkylsulfonic acid groups; and C atom in the alkyl groups may be optionally substituted by O atom, S atom, an aryl group or a heteroaryl group.

Further, R is selected from C1-C3 alkyl groups.

Further, m is 0 or 1.

Further, n is 0.

Further, X is selected from C1-C6 alkylene groups, and wherein C atom may be optionally substituted by O atom.

Further, Y is selected from C1-C7 alkylsulfonic acid groups or C1-C6 alkyl groups, and C atom in the alkyl groups may be optionally substituted by O atom.

Further, R is selected from C1-C2 alkyl groups.

Further, m is 0.

Further, X is selected from C3-C6 alkylene groups, and wherein C atom may be optionally substituted by O atom.

Further, Y is selected from C2-C4 alkylsulfonic acid groups or C1-C5 alkyl groups, and C atom in the alkyl groups may be optionally substituted by O atom.

Further, the luminescent label is at least one selected from the following compounds:

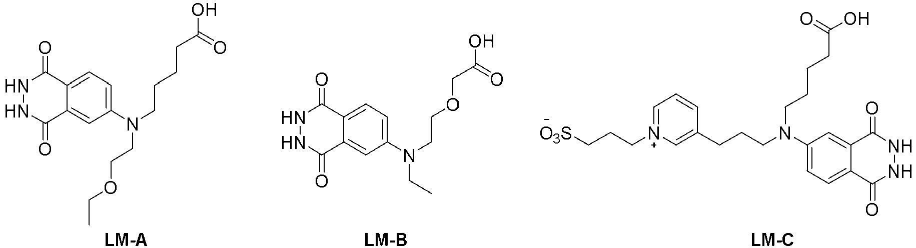

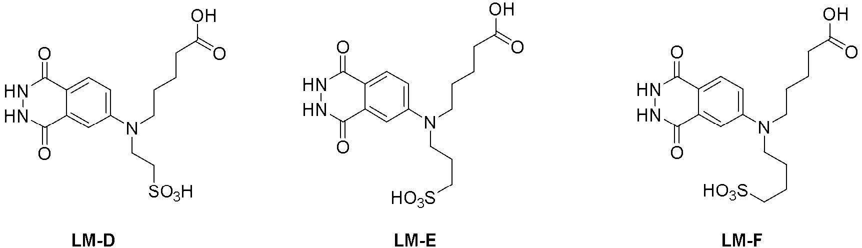

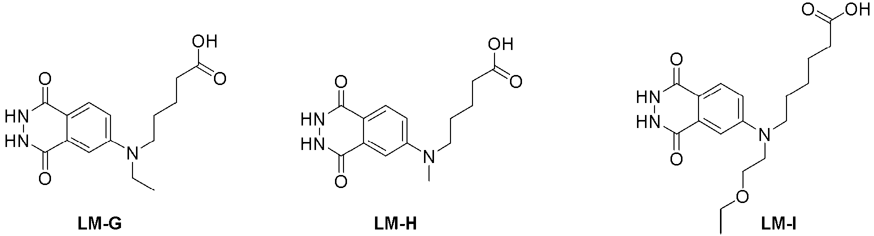

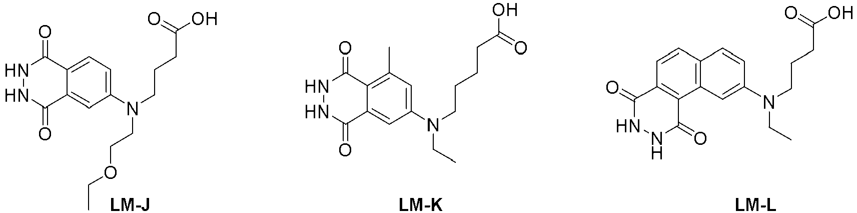

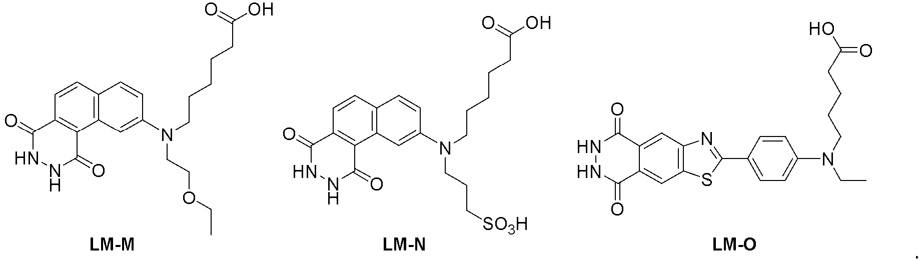

According to a second aspect of the present disclosure, provided is a luminescent label intermediate solution. Components of the luminescent label intermediate solution comprise a luminescent label intermediate and an organic solvent, wherein the luminescent label intermediate is formed by activating the luminescent label provided by the first aspect via an activator.

Further, the activator comprises an amide condensation agent.

Further, the amide condensation agent includes at least one of 1-(3-dimethylaminopropyl)-3-ethylcarbodiimide hydrochloride or dicyclohexylcarbodiimide.

Further, the activator further comprises N-hydroxysuccinimide, and the luminescent label intermediate has a structure as represented by the following formula (V):

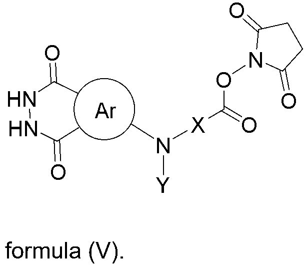

According to a third aspect of the present disclosure, further provided is a preparation method for the described luminescent label intermediate solution. The preparation method comprises: dispersing a luminescent label in an organic solvent, and adding an activator for an activation reaction to activate the luminescent label by the activator to form a luminescent label intermediate, so as to obtain a luminescent label intermediate solution.

Further, the activator comprises an amide condensation agent and optionally N-hydroxysuccinimide.

Further, the molar ratio of N-hydroxysuccinimide to the luminescent label is ≥1, and preferably (1.2-4):1.

Further, the temperature of the activation reaction is 10-50°C, and the time of the activation reaction is 8-20 h.

According to a fourth aspect of the present disclosure, further provided is use of the luminescent label provided by the first aspect or the luminescent label intermediate solution provided by the second aspect in preparation of a luminescent label conjugate.

According to a fifth aspect of the present disclosure, further provided is a luminescent label conjugate. The luminescent label conjugate is formed by coupling the luminescent label provided by the first aspect and a protein. The luminescent label conjugate has a structure as represented by the following formula (VI):

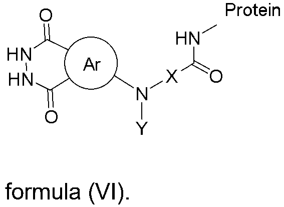

According to a sixth aspect of the present disclosure, further provided is a preparation method for the described luminescent label conjugate. The preparation method comprises: mixing the luminescent label intermediate solution provided by the second aspect with a protein to perform a coupling reaction, so as to obtain the luminescent label conjugate.

According to a seventh aspect of the present disclosure, further provided is use of the luminescent label provided in the described first aspect, the luminescent label intermediate solution provided in the described second aspect or the luminescent label conjugate provided in the described fifth aspect in luminescent immunoassay.

According to an eighth aspect of the present disclosure, further provided is a kit comprising the luminescent label provided in the described first aspect, the luminescent label intermediate solution provided in the described second aspect, or the luminescent label conjugate provided in the described fifth aspect.

By applying the technical solution provided in the present disclosure, the luminescent label provided in the present disclosure not only has good performance of luminescence intensity, but also can directly couple with a protein to generate a luminescent label intermediate under the action of an activator. The labeling process is simple, and the steric hindrance is lower during labeling, which can effectively improve the labeling efficiency.

### Detailed Description of the Embodiments

It is important to note that the examples of the present disclosure and the characteristics in the examples can be combined under the condition of no conflicts. Hereinafter, the present disclosure will be described in detail with reference to examples.

As analyzed in the background art of the present disclosure, the labels widely used at present are conjugate in labeling processes, affecting the labeling efficiency in practical application processes. In order to solve the problem, the present disclosure provides a luminescent label, a luminescent label intermediate solution and a preparation method therefor, a luminescent label conjugate, applications thereof, and a kit.

In a first typical embodiment of the present disclosure, provided is a luminescent label. The luminescent label has a structure as represented by general formula (I): 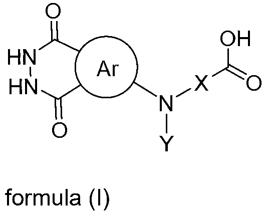
wherein Ar is any one selected from general formulas (II)-(IV): 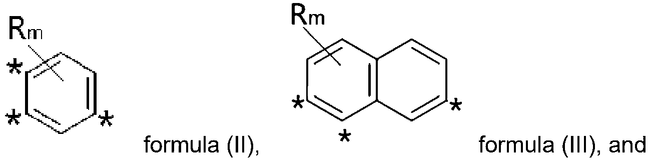 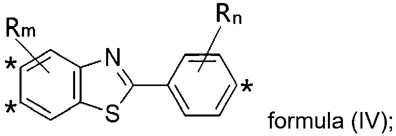
the described Rm refers to m instances of R, and the described Rn refers to n instances of R; m is an integer between 0 and 2, such as 0, 1, or 2, and when m = 2, each R is the same or different; n is an integer between 0 and 4, and when n ≥ 2, each R is the same or different; R is selected from C1-C10 alkyl groups; X is selected from C1-C10 alkylene groups, and C atom or H atom thereof may be optionally substituted by O atom, S atom, an aryl group and a derivative thereof or a heteroaryl group and a derivative thereof; Y is selected from C1-C10 alkyl groups or C1-C10 alkylsulfonic acid groups; and C atom in the alkyl groups may be optionally substituted by O atom, S atom, an aryl group and a derivative thereof or a heteroaryl group and a derivative thereof.

In the present disclosure, "aryl groups" represent aryl groups or arylene groups, and the aryl groups refer to monocyclic or fused polycyclic rings derived from aromatic hydrocarbons, and include phenyl, biphenyl, terphenyl, naphthyl, binaphthyl, phenylnaphthyl, naphthylphenyl, fluorenyl, phenylfluorenyl, benzofluorenyl, dibenzofluorenyl, phenanthrenyl, phenylphenanthrenyl, anthracenyl, indenyl, bistriphenylenyl, pyrenyl, fused tetraphenyl, perylenyl, chrysenyl, naphthacenyl, propadienefluorenyl, and the like.

"Heteroaryl groups" refer to heteroaryl groups or heteroarylene groups, and refer to aryl groups having at least one heteroatom selected from the group consisting of N, O, and S as a ring backbone atom, and may be monocyclic rings such as furyl, thienyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, and pyridazinyl, or condensed with at least one benzene ring, such as benzofuranyl, benzothienyl, isobenzofuranyl, dibenzofuranyl, dibenzothienyl, benzimidazolyl, benzothiazolyl, benzoisothiazolyl, benzoisoxazolyl, quinolinyl, isoquinolinyl, cinnolinyl, quinazolinyl, quinoxalinyl, carbazolyl, phenanthrenoxazolyl, phenanthridinyl, benzodiacenaphthenyl, and dihydroacridinyl.

"C1-C10 alkyl groups" refer to alkyl groups having 1 to 10, in particular 6 carbon atoms, and the groups are straight chains or branched chains having single or multiple branches, for example butyl, such as n-butyl, sec-butyl, isobutyl, or tert-butyl; propyl, such as n-propyl or isopropyl; and ethyl or methyl; more particularly methyl, isopropyl or tert-butyl.

"C1-C10 alkylsulfonic acid groups" refer to "C1-C10 alkyl-SO₃H", in particular ethylsulfonic acid, butylsulfonic acid, pentylsulfonic acid, hexylsulfonic acid and the like.

The luminescent label provided in the present disclosure is obtained by means of a series of structural designs. The luminescent label not only has good performance of luminescent intensity, but also can directly couple with a protein to generate a luminescent label intermediate under the action of an activator. The labeling process is simple, and the steric hindrance is lower during labeling, which can effectively improve the labeling efficiency while ensuring the luminescent performance.

In some examples, R is selected from C1-C6 alkyl groups.

In some examples, X is selected from C1-C6 alkylene groups, and C atom thereof may be optionally substituted by O atom, which is more beneficial to improving labeling efficiency and luminescent intensity.

In some examples, Y is selected from C1-C7 alkylsulphonic acid groups or C1-C6 alkyl groups, and C atom in the alkyl groups may be optionally substituted by O atom, which is more beneficial to improving labeling efficiency and luminescent intensity.

In some examples, X is selected from the group consisting of C3-C6 alkylene groups, and wherein C atom may be optionally substituted by O atom.

In some examples, Y is selected from C2-C4 alkylsulfonic acid groups or C1-C5 alkyl groups, and C atom in the alkyl groups may be optionally substituted by O atom, which is more beneficial to improving labeling efficiency and luminescent intensity.

In some specific examples, the luminescent label is at least one selected from:

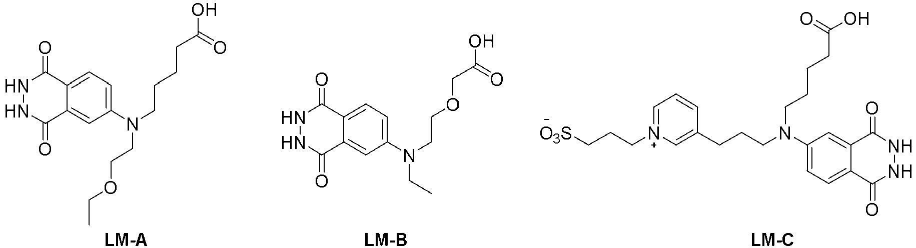

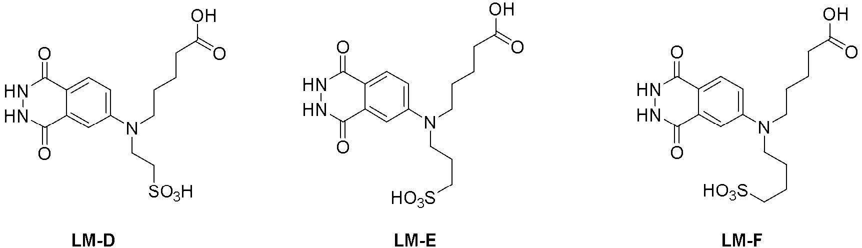

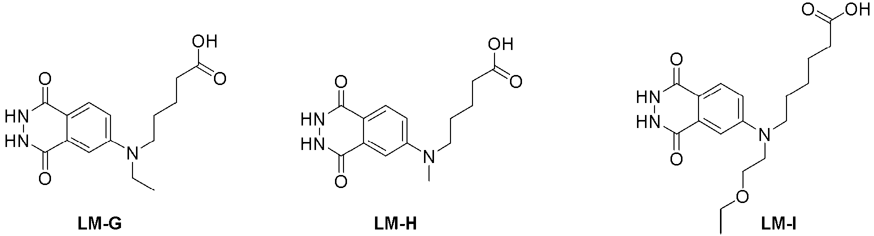

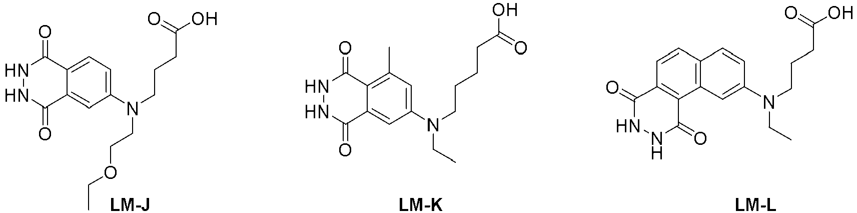

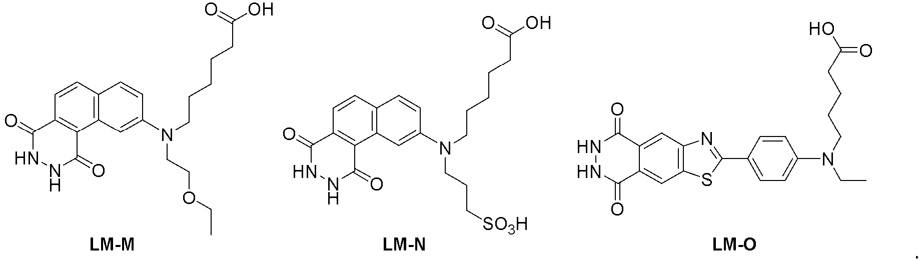

In the present disclosure, the luminescent label is prepared according to a conventional method in the art, which is not limited herein.

In some examples, taking a luminescent label LM-A as an example, the preparation of the luminescent label and the activation process of the luminescent label are as shown in (A).

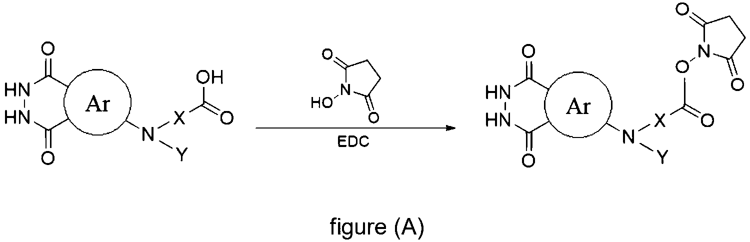

In some examples, the synthetic pathway for a luminescent label TM-A is as shown in (B) below.

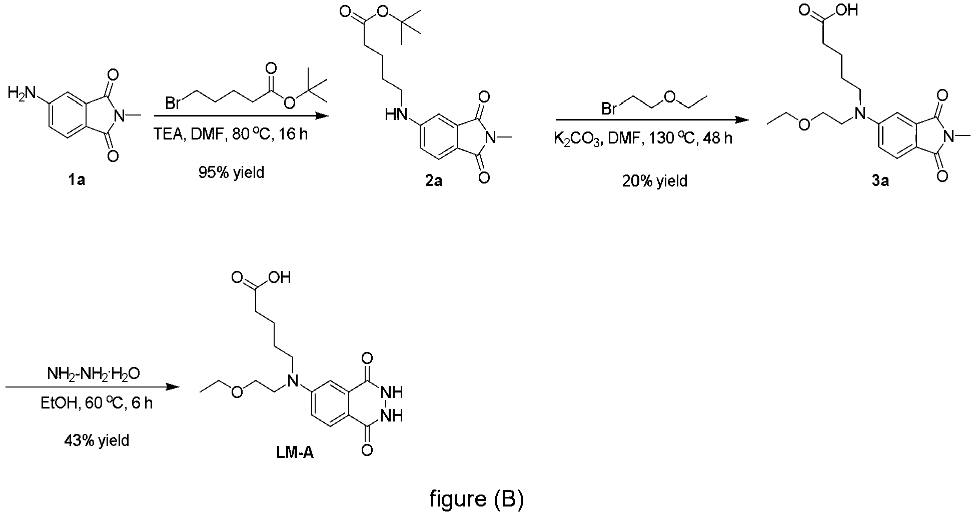

Specifically, the luminescent label TM-A was prepared according to the following steps:

### (1) Preparation of compound 2a.

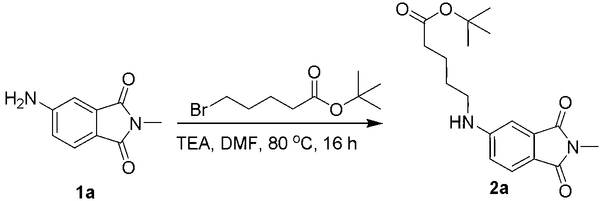

To a Schlenk tube was added compound 1a (3 g, 17.03 mmol), tert-butyl 5-bromovalerate (20.2 g, 85.15 mmol), triethylamine (8.6 g, 85.15 mmol) and DMF (45 mL). Under argon protection, the mixture was heated at 80 °C for 20 hours. The reaction was monitored by LC-MS until the starting material was mostly converted to the desired product. After completion, the crude mixture was roughly purified by reverse-phase preparative chromatography on a C18 column using CH₃CN/H₂O (0.1% FA) as the mobile phase. The fractions containing compound **2a** were collected, concentrated by rotary evaporation to remove acetonitrile, and the aqueous layer was extracted with ethyl acetate. The combined organic extracts were dried and concentrated to afford crude compound **2a** (5.37 g, 95% yield), which was used directly in the next step. MS [M-(t-BuO-)]⁺: calcd. 259.11, found 259.0.

The described "FA" refers to "Formic Acid"; "LS-MS" refers to "Liquid Chromatography-Mass Spectrometer".

### (2) Preparation of Compound 3a.

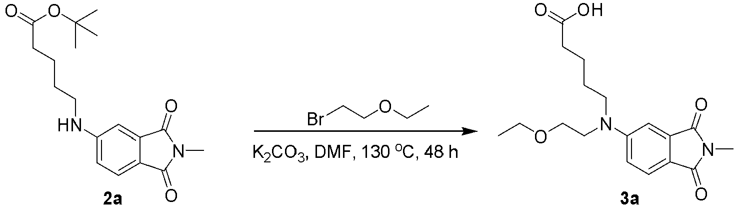

Compound **2a** (1.0 g, 3.01 mmol), DMF (10 mL), 2-bromoethyl ethyl ether (4.6 g, 30.1 mmol) and potassium carbonate (1.25 g, 9.03 mmol) were added into a sealed tube. The mixture was stirred at 130 °C for 48 hours, after that the conversion of starting material was about 20% by LC-MS detection. The reaction was then stopped, the crude mixture was roughly purified by reversed-phase preparative chromatography on a C18 column using CH₃CN/H₂O (0.1% FA) as the mobile phase, which offered a solution of compound **3a.** Acetonitrile was removed by rotary evaporation, and the aqueous phase was extracted with dichloromethane. The resulting organic phase was then evaporated to dryness to give crude compound **3a.** The crude product was further purified by silica gel column chromatography, finally yielding pure compound **3a** (209.6 mg, 20% yield). MS [M+H]⁺: calcd. 349.17, found 349.0.

### (3) Preparation of LM-A.

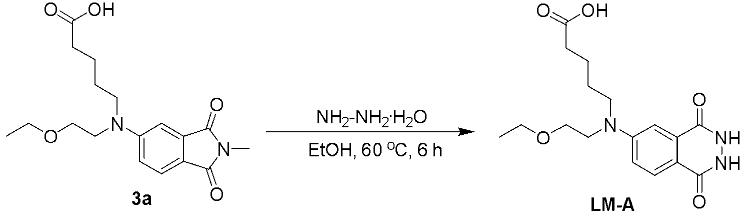

To a Schlenk tube, compound **3a** (200 mg, 0.574 mmol), ethanol (20 mL) and hydrazine hydrate (574 mg, 11.5 mmol) were added. The mixture was heated and stirred at 60 °C for 6 hours. After the reaction was completed, the solvent and excess hydrazine hydrate were removed by vacuum rotary evaporation to obtain a crude product. 3 N hydrochloric acid was added dropwise to the crude product until it became acidic (pH = 1-2), stirring should be maintained during the neutralization process, and the pH endpoint should remain stable for more than 10 minutes. Water and acetonitrile were added to dissolve the sample, and the crude solution was directly used for reversed-phase purification. Purification was performed on a reversed-phase C18 column using CH₃CN/H₂O (0.1% FA) as the mobile phase, yielding pure compound **LM-A** (86 mg, 43% yield). ¹H NMR (400 MHz, DMSO) δ 11.42 (s, 3H), 7.82 (d, J = 8.9 Hz, 1H), 7.21 (dd, J = 9.1, 2.8 Hz, 1H), 7.06 (s, 1H), 3.62 - 3.53 (m, 4H), 3.47 - 3.41 (m, 4H), 2.29 - 2.23 (m, 2H), 1.56 (dd, J = 7.0, 4.0 Hz, 4H), 1.09 (t, J = 7.0 Hz, 3H). MS [M+H]⁺: calcd. 350.16, found 350.0.

According to a second typical embodiment of the present disclosure, a luminescent label intermediate solution is provided. Components of the luminescent label intermediate solution comprise a luminescent label intermediate and an organic solvent, wherein the luminescent label intermediate is formed by activating the luminescent label provided by the first typical embodiment by means of an activator.

In the present disclosure, the "luminescent label intermediate" refers to an activated state of the described luminescent label, and the coupling efficiency of the luminescent label and a protein can be further improved after activating the luminescent label into the activated state, wherein the activated state can be a luminescent label activated ester, and the stability of the activated state of the luminescent label can be improved after preparing the luminescent label into a luminescent label activated ester.

"Organic solvents" refer to a class of solvents with an organic substance as a medium, it is generally characterized by a relatively low molecular weight, a liquid state under standard ambient conditions, high volatility, and the ability to dissolve various water-insoluble organic compounds. There are many types of common organic solvents, and the organic solvents can be classified into more than ten major classes according to chemical structures including: aromatic hydrocarbons (such as benzene and toluene), aliphatic hydrocarbons (such as pentane and hexane), halogenated hydrocarbons (such as chlorobenzene and dichloromethane), alcohols (such as methanol and ethanol), ethers (such as diethyl ether), esters (such as methyl acetate) and ketones (such as acetone).

The described activators are commonly used activators for activating carboxyl group, and are not limited in the present disclosure.

In some embodiments, the activator comprises an amide condensation agent.

In the present disclosure, "amide condensation agent" refers to agents capable of activating a luminescent label into an activated state, which can accelerate and promote a condensation reaction between a carboxylic acid and an amine to form an amide bond. Common amide condensation agents include 1-(3-dimethylaminopropyl)-3-ethylcarbodiimide hydrochloride (EDC), dicyclohexylcarbodiimide (DCC), O-(1H-Benzotriazol-1-yl)-N,N,N',N'-tetramethyluronium hexafluorophosphate (HBTU), diisopropylcarbodiimide (DIC), and alkynamide.

In some embodiments, when the activator further comprises N-hydroxysuccinimide, the luminescent label intermediate has a structure as represented by formula (V):

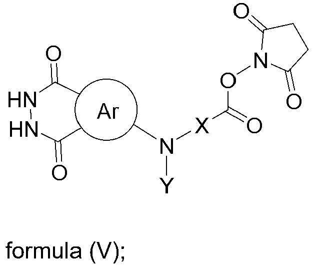

In the described formula (V), Ar, X, and Y each have the same meanings as in the luminescent label provided in the first typical embodiment, which are not described in detail here.

In some examples, the organic solvent provides a reaction environment of the luminescent label and N-hydroxysuccinimide, and is a common organic solvent in the art, including but not limited to DMSO (dimethyl sulfoxide), DMF (dimethylformamide), etc.

In some examples, the activation of the carboxyl group of LM-A to form a luminescent label intermediate LM-A-S is performed as follows:

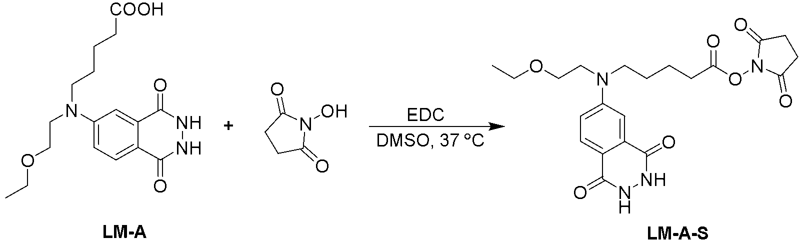

Compound **LM-A** (115 mg, 0.328 mmol) was dissolved in DMSO (5 mL), followed by addition of EDC (125.6 mg, 0.655 mmol). After stirring at room temperature under argon protection for 20 minutes, NHS (75.4 mg, 0.655 mmol) was added, and the mixture was stirred at 40°C for 12 hours. After completion of the reaction, compound **LM-A-S** was first isolated by reversed-phase C18 column chromatography using CH₃CN/H₂O (0.1% TFA) as the mobile phase, and then lyophilized to obtain compound **LM-A-S** (53 mg, 36% yield). MS [M+H]⁺: calcd. 447.2, found 447.3.

According to a third typical embodiment of the present disclosure, provided is a preparation method for the described luminescent label intermediate solution. The preparation method comprises: dispersing a luminescent label in an organic solvent, adding an activator and mixing same to perform an activation reaction, so that the luminescent label is activated by the activator to form a luminescent label intermediate, so as to obtain a luminescent label intermediate solution.

The meaning of the activator is the same as that described in the second typical embodiment, and is not described herein.

In some examples, the activator comprises an amide condensation agent and N-hydroxysuccinimide, and the molar ratio of N-hydroxysuccinimide to the luminescent label is ≥ 1, a molar ratio of (1.2-4):1 is especially beneficial as it improves the labeling efficiency while saving energy.

In particular, the molar ratio of N-hydroxysuccinimide to the luminescent label may be 1.2:1, 1.5:1, 2:1, 2.5:1, 3:1, 3.5:1, 4:1, or in a range formed by any two values.

In order to further improve the reaction efficiency, in some examples, the temperature of the activation reaction is 10-50°C, and the time of the activation reaction is 8-20 h. Specifically, the temperature of the activation reaction may be 10°C, 15°C, 20°C, 25°C, 30°C, 35°C, 40°C, 45°C, 50°C or in a range formed by any two values; and the time of the activation reaction may be 8 h, 10 h, 12 h, 15 h, 18 h, 20 h, or in a range formed by any two values.

In a fourth typical embodiment of the present disclosure, further provided is use of the luminescent label provided by the described first typical embodiment or the luminescent label intermediate solution provided by the second typical embodiment in preparation of a luminescent label conjugate.

In the present disclosure, a luminescent label conjugate is formed by coupling the luminescent label and a protein, and the luminescent label conjugate has a structure as represented by formula (VI) below:

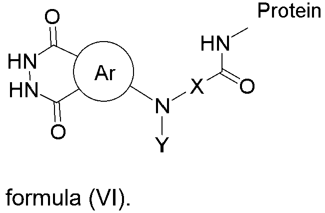

"Protein" refers to a protein comprising an amino group, and is coupled to the luminescent label intermediate via an amino group to form a structure as represented by formula (VI).

In the described formula (VI), Ar, X, and Y each have the same meaning as those in the luminescent label provided in the first typical embodiment, which are not described in detail here.

In some examples, the activator further comprises N-hydroxysuccinimide, and the luminescent label intermediate has a structure as represented by formula (V):

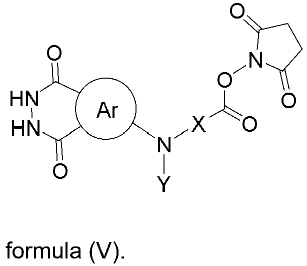

In a third typical embodiment of the present disclosure, provided is a luminescent label intermediate solution, the components of which comprise the luminescent label intermediate provided in the second typical embodiment and an organic solvent.

In a fourth typical embodiment of the present disclosure, further provided is a preparation method for the luminescent label activating intermediate solution. The preparation method comprises: dispersing a luminescent label in an organic solvent, adding an amide condensation agent and N-hydroxysuccinimide and mixing same for a reaction, so as to obtain a luminescent label intermediate solution. The luminescent label intermediate in the luminescent label intermediate solution may be stored stably.

In some examples, in the luminescent label activating intermediate solution provided in the present disclosure, the amide condensation agent is selected from EDC or DCC, and the described amide condensation agent can stably and efficiently react with the luminescent label, and the reaction conditions are mild.

According to fifth typical embodiment of the present disclosure, further provided is a luminescent label conjugate. The luminescent label conjugate is formed by coupling the luminescent label and a protein. The luminescent label conjugate has a structure as represented by the following formula (VI):

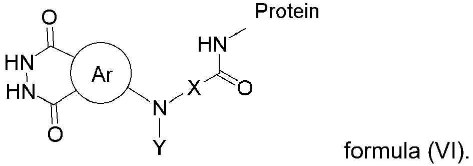

Exemplarily, the reaction principle of coupling the luminescent label intermediate with a protein (an amino-containing protein) is as shown in figure (C):

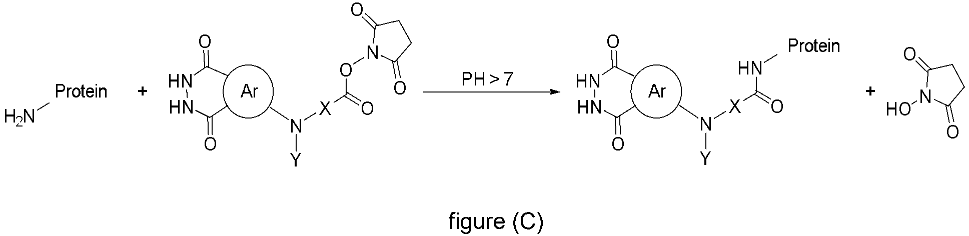

According to the sixth typical embodiment of the present disclosure, provided is use of the described luminescent label, the described luminescent label intermediate solution, or the described luminescent label conjugate in luminescent immunoassay.

The luminescent label, the luminescent label intermediate solution, or the luminescent label conjugate provided in the present disclosure is applied in the field of luminescent immunoassay, and are more conducive to improving the labeling efficiency, and has wide application prospects.

According to the seventh typical embodiment of the present disclosure, further provided is a kit comprising the described luminescent label, luminescent label intermediate solution, or luminescent label conjugate.

The kit provided in the present disclosure uses the luminescent label as a main raw material and utilizes a carboxyl group on the luminescent label to couple with an amino group on the protein to form the luminescent label conjugate. The labeling process is simple, the labeling efficiency is improved effectively, and the kit has wide applications in the field of luminescent immunoassay.

Beneficial effects of the present disclosure will be further described below in conjunction with the examples and comparative examples.

### Example 1

The synthetic route of **LM-A** is shown as follows:

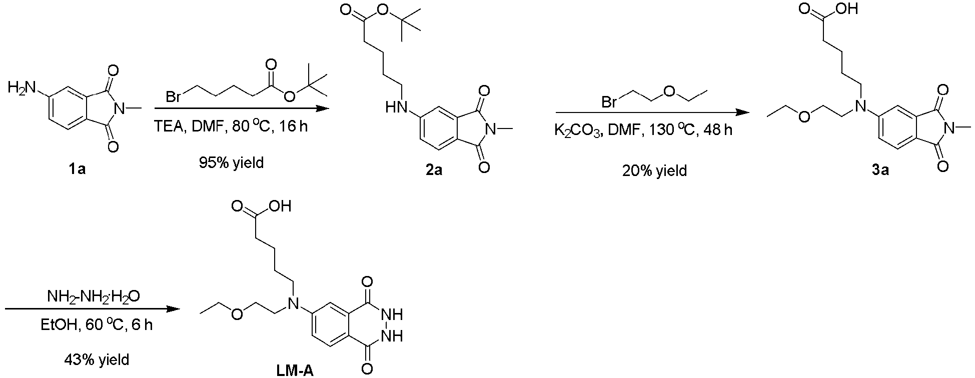

### (1) Preparation of compound 2a.

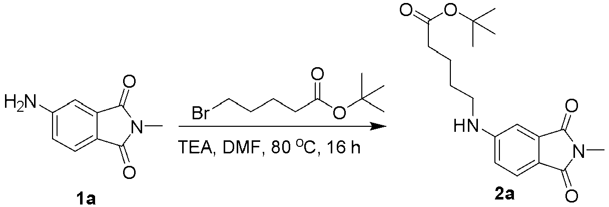

To a Schlenk tube was added compound **1a** (3 g, 17.03 mmol), tert-butyl 5-bromovalerate (20.2 g, 85.15 mmol), triethylamine (8.6 g, 85.15 mmol) and DMF (45 mL). Under argon protection, the mixture was heated at 80 °C for 20 hours. The reaction was monitored by LC-MS until the starting material was mostly converted to the desired product. After completion, the crude mixture was roughly purified by reverse-phase preparative chromatography on a C18 column using CH₃CN/H₂O (0.1% FA) as the mobile phase. The fractions containing compound **2a** were collected, concentrated by rotary evaporation to remove acetonitrile, and the aqueous layer was extracted with ethyl acetate. The combined organic extracts were dried and concentrated to afford crude compound **2a** (5.37 g, 95% yield), which was used directly in the next step. MS [M-(t-BuO-)]⁺: calcd. 259.11, found 259.0.

### (2) Preparation of compound 3a.

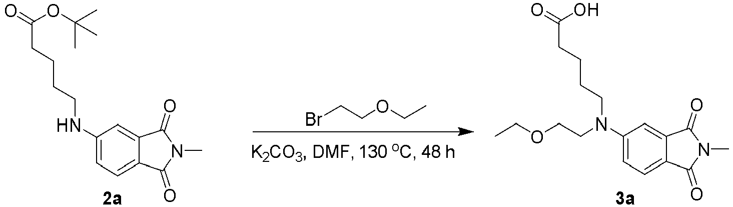

Compound **2a** (1.0 g, 3.01 mmol), DMF (10 mL), 2-bromoethyl ethyl ether (4.6 g, 30.1 mmol) and potassium carbonate (1.25 g, 9.03 mmol) were added into a sealed tube. The mixture was stirred at 130 °C for 48 hours, after that the conversion of starting material was about 20% by LC-MS detection. The reaction was then stopped, the crude mixture was roughly purified by reversed-phase preparative chromatography on a C18 column using CH₃CN/H₂O (0.1% FA) as the mobile phase, which offered a solution of compound **3a.** Acetonitrile was removed by rotary evaporation, and the aqueous phase was extracted with dichloromethane. The resulting organic phase was then evaporated to dryness to give crude compound **3a.** The crude product was further purified by silica gel column chromatography, finally yielding pure compound **3a** (209.6 mg, 20% yield). MS [M+H]⁺: calcd. 349.17, found 349.0.

### (3) Preparation of LM-A.

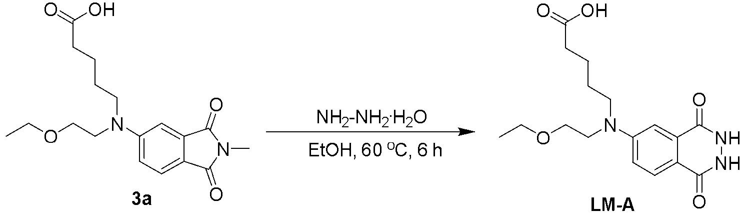

To a Schlenk tube, compound 3a (200 mg, 0.574 mmol), ethanol (20 mL) and hydrazine hydrate (574 mg, 11.5 mmol) were added. The mixture was heated and stirred at 60 °C for 6 hours. After the reaction was completed, the solvent and excess hydrazine hydrate were removed by vacuum rotary evaporation to obtain a crude product. 3 N hydrochloric acid was added dropwise to the crude product until it became acidic (pH = 1-2), stirring should be maintained during the neutralization process, and the pH endpoint should remain stable for more than 10 minutes. Water and acetonitrile were added to dissolve the sample, and the crude solution was directly used for reversed-phase purification. Purification was performed on a reversed-phase C18 column using CH₃CN/H₂O (0.1% FA) as the mobile phase, yielding pure compound **LM-A** (86 mg, 43% yield). ¹H NMR (400 MHz, DMSO) δ 11.42 (s, 3H), 7.82 (d, J = 8.9 Hz, 1H), 7.21 (dd, J = 9.1, 2.8 Hz, 1H), 7.06 (s, 1H), 3.62 - 3.53 (m, 4H), 3.47 - 3.41 (m, 4H), 2.29 - 2.23 (m, 2H), 1.56 (dd, J = 7.0, 4.0 Hz, 4H), 1.09 (t, J = 7.0 Hz, 3H). MS [M+H]⁺: calcd. 350.16, found 350.0.

### Carboxyl Activation of LM-A.

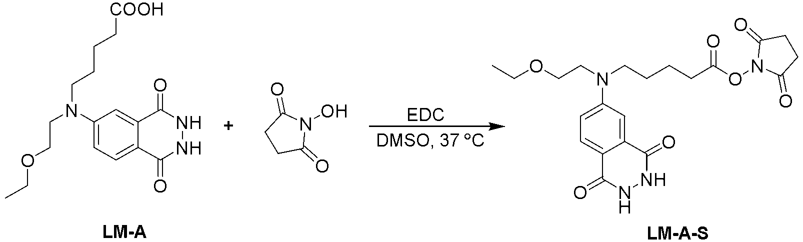

Compound **LM-A** (115 mg, 0.328 mmol) was dissolved in DMSO (5 mL), followed by the addition of EDC (125.6 mg, 0.655 mmol). After stirring at room temperature under argon protection for 20 minutes, NHS (75.4 mg, 0.655 mmol) was added, and the mixture was stirred at 40°C for 12 hours. After completion of the reaction, compound **LM-A-S** was first isolated by reversed-phase C18 column chromatography using CH₃CN/H₂O (0.1% TFA) as the mobile phase, and then lyophilized to obtain compound **LM-A-S** (53 mg, 36% yield). MS [M+H]⁺: calcd. 447.2, found 447.3.

### Example 2

The synthetic route of **LM-B** is shown as follows:

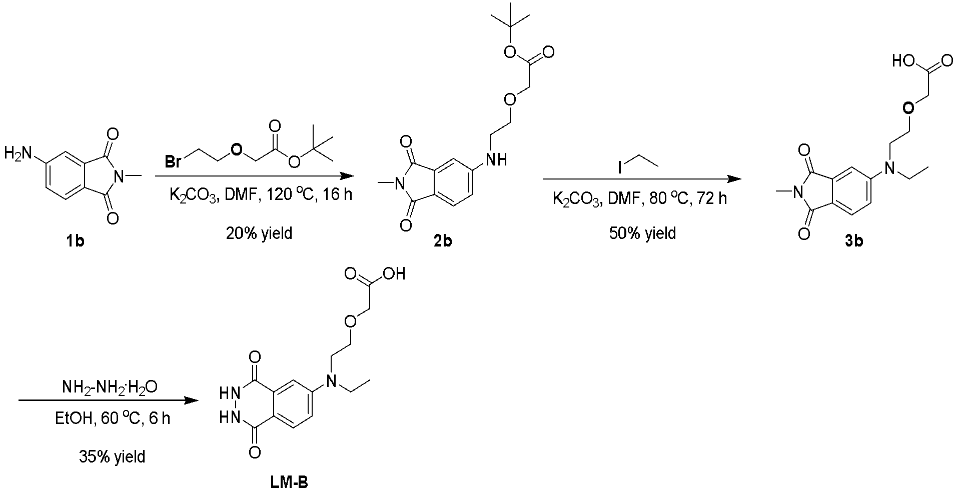

### (1) Preparation of compound 2b.

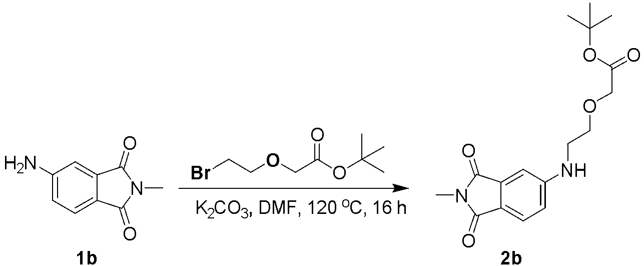

To a Schlenk tube was added compound 1b (300 mg, 1.7 mmol), tert-butyl 2-(2-bromoethoxy)acetate (1.02 g, 4.25 mmol), potassium carbonate (704.8 mg, 5.1 mmol) and DMF (10 mL). The tube was flushed with argon and the mixture was heated at 120 °C for 16 hours. The reaction was monitored by TLC (PE:EA = 2:1, Rf = 0.6). After completion, the mixture was purified by silica gel column chromatography. The fractions containing the product were collected and concentrated by rotary evaporation to afford pure compound **2b** (113.5 mg, 20% yield). MS [M-(t-BuO-)]⁺: calcd. 261.09, found 262.1.

### (2) Preparation of compound 3b.

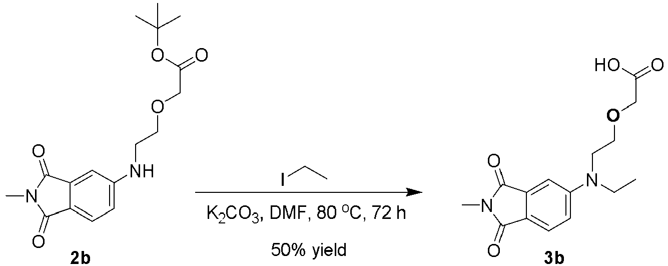

Compound **2b** (200 mg, 0.598 mmol), potassium carbonate (248 mg, 1.79 mmol), iodoethane (1.87 g, 11.96 mmol) and DMF (4 mL) were added to a sealed tube. The mixture was stirred at 80°C for 72 hours, after that approximately 50% conversion of the starting material was observed by LC-MS analysis. The reaction was stopped, the crude mixture was purified by reverse-phase preparative chromatography on a C18 column using CH₃CN/H₂O (0.1% FA) as the mobile phase. The fractions containing compound **3b** were concentrated by rotary evaporation to remove acetonitrile, and the remaining aqueous solution was lyophilized to afford pure compound **3b** (91.5 mg, 50% yield). MS [M+H]⁺: calcd. 307.12, found 307.0.

### (3) Preparation of LM-B.

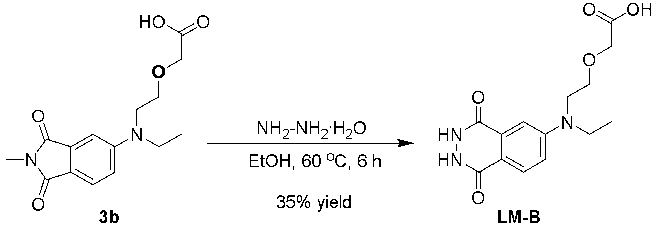

To a Schlenk tube was added compound **3b** (90 mg, 0.294 mmol), ethanol (2 mL), and hydrazine hydrate (294 mg, 5.88 mmol). The mixture was heated and stirred at 60 °C for 6 hours. After the reaction was completed, the solvent and excess hydrazine hydrate were removed by vacuum rotary evaporation to obtain a crude product. 3N hydrochloric acid was added dropwise to the crude product until it became acidic (pH = 1-2), stirring should be maintained during the neutralization process, and the pH endpoint should remain stable for more than 10 minutes. Water and acetonitrile were added to dissolve the sample, and the crude solution was directly used for reversed-phase purification. Purification was performed on a reversed-phase C18 column using CH₃CN/H₂O (0.1% FA) as the mobile phase to obtain pure compound **LM-B** (31.6 mg, 35% yield). ¹H NMR (400 MHz, DMSO) δ 7.83 (d, J = 8.9 Hz, 1H), 7.22 (dd, J = 9.1, 2.7 Hz, 1H), 7.06 (d, J = 2.7 Hz, 1H), 4.04 (s, 2H), 3.66 (d, J = 4.8 Hz, 2H), 3.62 (d, J = 4.9 Hz, 2H), 3.51 (q, J = 7.0 Hz, 2H), 1.14 (t, J = 7.0 Hz, 3H). MS [M+H]⁺: calcd. 308.12, found 308.0.

The activation step was performed following the same procedure as for **LM-A.**

### Example 3

The synthetic route of **LM-C** is shown as follows:

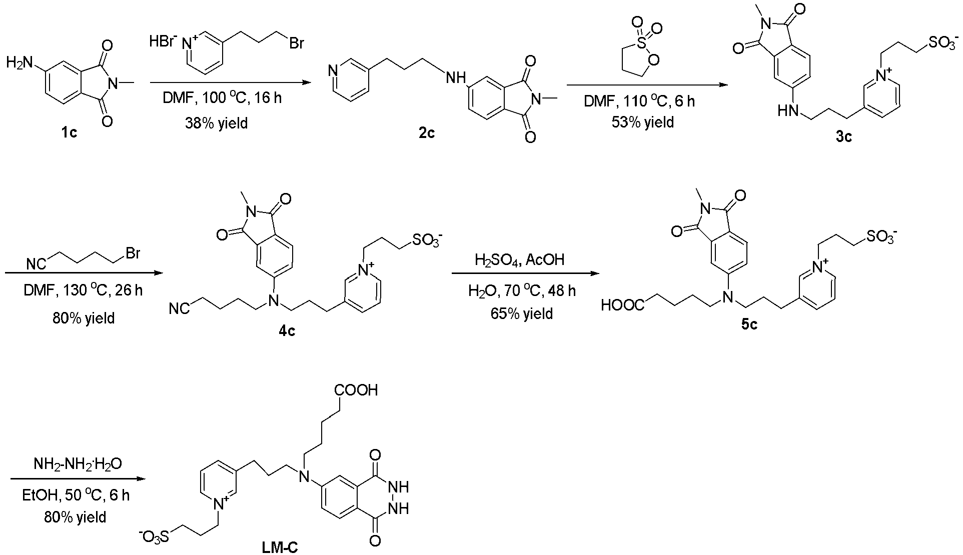

### (1) Preparation of compound 2c.

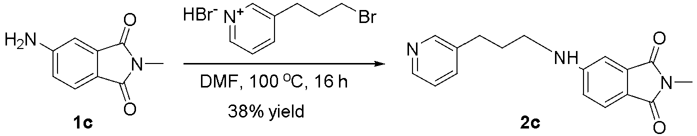

Compound 1c (500 mg, 2.8 mmol), 3-bromopropylpyridinium hydrobromide (1.5 g, 5.3 mmol), and DMF (30 mL) were added to a three-necked flask. Under argon protection, the mixture was heated at 100°C for 16 hours. LC-MS monitoring indicated that the starting material was mostly consumed. After cooling to room temperature, the crude product was purified by reverse-phase chromatography on a C18 column using CH₃CN/H₂O (0.1% FA) as the mobile phase to afford pure compound **2c** (300 mg, 38% yield). MS [M+H]⁺: calcd. 296.13, found 296.4.

### (2) Preparation of compound 3c.

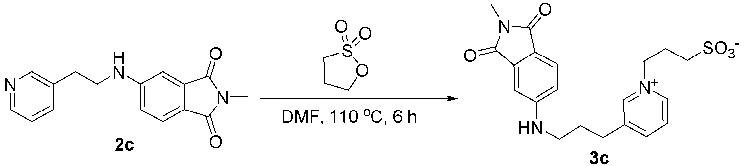

Compound **2c** (1.0 g, 3.6 mmol), 1,3-propanesultone (0.82 g, 6.0 mmol) and DMF (40 mL) were added to a Schlenk tube. Under argon protection, the mixture was heated at 110 °C for 6 hours. LC-MS monitoring showed that the starting material was mostly consumed. After completion, the reaction mixture was cooled to room temperature and purified by reverse-phase chromatography on a C18 column using CH₃CN/H₂O (0.1% FA) as the mobile phase to obtain pure compound **3c** (797 mg, 53% yield). MS [M+H]⁺: calcd. 418.14, found 418.1.

### (3) Preparation of compound 4c.

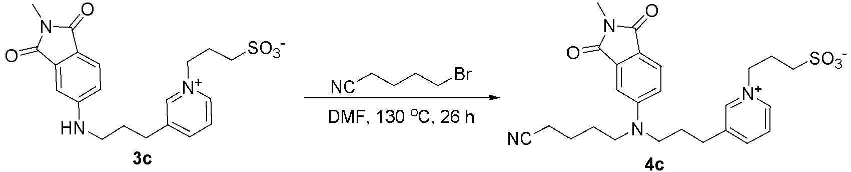

Compound 3c (500 mg, 1.20 mmol), pentabromovaleronitrile (1.94 g, 12.0 mmol), and DMF (5 mL) were added to a sealed tube and heated at 130°C for 26 hours. LC-MS analysis indicated complete conversion of the starting material. After cooling to room temperature, the crude product was purified by reverse-phase chromatography on a C18 column using CH₃CN/H₂O (0.1% FA) as the mobile phase to afford pure compound 4c (479 mg, 80% yield). MS [M+H]⁺: calcd. 499.19, found 499.2.

### (4) Preparation of compound 5c.

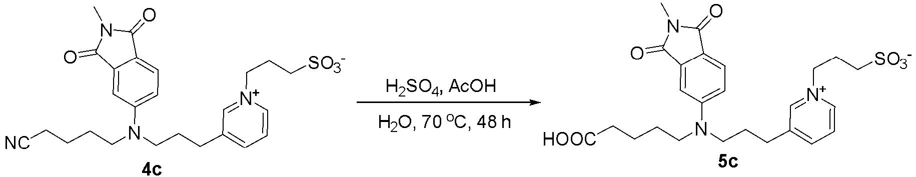

To a glass sealed tube were added H₂O (0.9 mL), glacial acetic acid (0.9 mL) and concentrated sulfuric acid (1.8 mL) in sequence. After thorough mixing, compound **4c** (900 mg, 1.8 mmol) was added, and the reaction mixture was heated and stirred at 70 °C for 48 hours. The reaction was monitored by LC-MS and stopped when the conversion rate of the target product exceeded 85%. After completion, the reaction mixture was cooled to room temperature and diluted with H₂O (15 mL). The resulting clear solution was directly used for reversed-phase purification. Pure compound **5c** (606 mg, 65% yield) was obtained by purification on a reversed-phase C18 column using CH₃CN/H₂O (0.1% FA) as the mobile phase. MS [M+H]⁺: calcd. 518.19, found 518.2.

### (5) Preparation of compound LM-C.

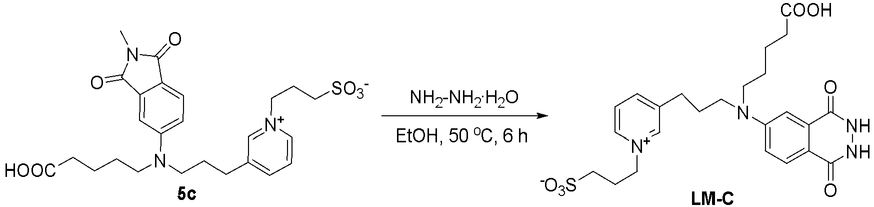

In a Schlenk tube, compound **5c** (500 mg, 0.97 mmol), ethanol (5 mL), and hydrazine hydrate (966.0 mg, 19.3 mmol) were added. The mixture was heated and stirred at 50 °C for 6 hours. After the reaction was completed, the solvent and excess hydrazine hydrate were removed by vacuum rotary evaporation to obtain a crude product. 3 N hydrochloric acid was added dropwise to the crude product to adjust the pH to 1-2 (stirring should be maintained during the neutralization process, and the pH endpoint should remain stable for more than 10 minutes). H₂O (10 mL) and acetonitrile (10 mL) were added to dissolve the sample, then the resulting crude solution was directly used for reverse-phase purification. Purification was performed on a reverse-phase C18 column with CH₃CN/H₂O (0.1% FA) as the mobile phase to obtain the pure compound **LM-C** (415 mg, 80% yield). 1H NMR (400 MHz, DMSO) δ 9.05 (s, 1H), 8.94 (d, J = 6.0 Hz, 1H), 8.50 (d, J = 7.9 Hz, 1H), 8.05 (dd, J = 8.0, 6.0 Hz, 1H), 7.82 (d, J = 9.0 Hz, 1H), 7.19 (dd, J = 9.1, 2.8 Hz, 1H), 6.96 (d, J = 2.7 Hz, 1H), 4.69 (t, J = 7.0 Hz, 2H), 3.48 (t, J = 7.6 Hz, 2H), 3.42 (t, J = 6.9 Hz, 2H), 2.89 (t, J = 7.7 Hz, 2H), 2.45 (t, J = 7.1 Hz, 2H), 2.25 (q, J = 7.1 Hz, 4H), 1.96 (p, J = 7.9 Hz, 2H), 1.56 (p, J = 3.4 Hz, 4H). MS [M+H]⁺: calcd. 519.18, found 519.2.

### Preparation of compound LM-C-S.

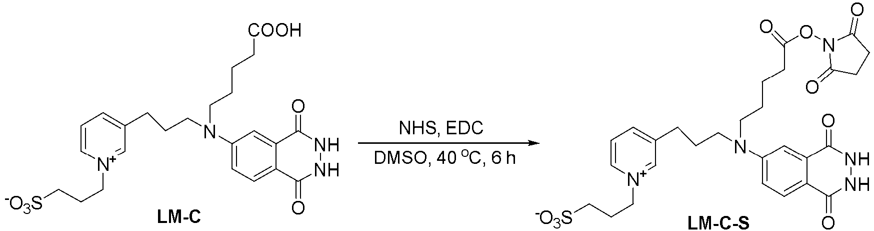

To a Schlenk tube, **LM-C** (100 mg, 0.192 mmol) and NHS (88 mg, 0.768 mmol) were added, followed by DMSO (5 mL). After purging with argon, the mixture was stirred at 40 °C for 5 minutes. Then, the entire amount of EDC (110 mg, 0.576 mmol) was quickly added to the reaction solution, and the mixture was stirred at 40 °C for 6 hours. The reaction was monitored by LC-MS and stopped until the conversion of product exceeded 70%. Upon completion of the reaction, DMSO solution of the chemiluminescent label (the active ester LM-C-S) was obtained, which could be directly used for protein labeling. MS [M+H]⁺: calcd. 616.20, found 616.5.

### Example 4

The synthetic route of **LM-D** is shown as follows:

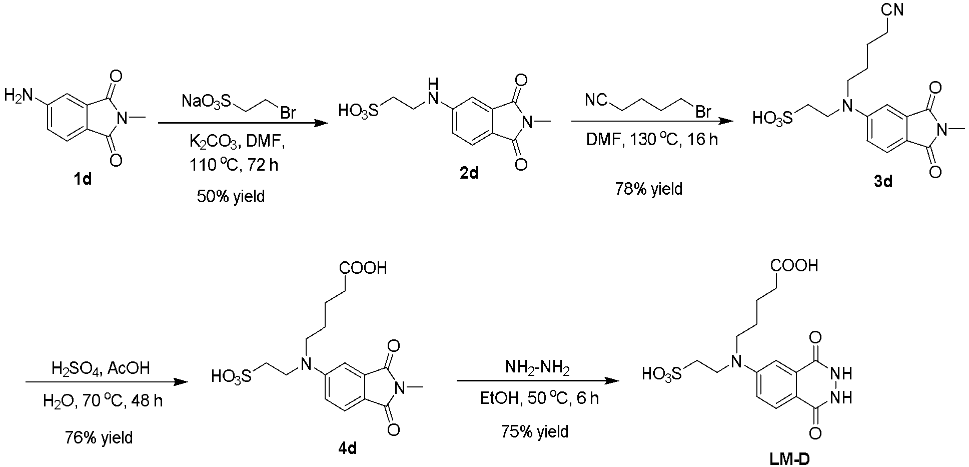

### (1) Preparation of compound 2d.

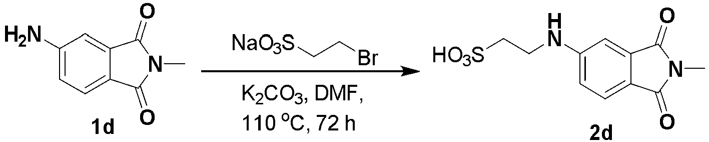

To a Schlenk tube, compound **1d** (2 g, 11.35 mmol), sodium 2-bromoethylsulfonate (24 g, 113.7 mmol), potassium carbonate (7.9 g, 57.2 mmol) and DMF (40 mL) were added. Under argon protection, the mixture was heated at 110 °C for 72 hours. The reaction solution was monitored by LC-MS, and reaction was stopped until the conversion of product exceeded 70%. After the reaction solution was cooled to room temperature, H₂O was added to the system and ultrasonic oscillation was performed until the solution became clear. The crude solution was purified by reversed-phase preparation using a C18 column with CH₃CN/H₂O (0.1% FA) as the mobile phase to obtain a solution of compound 2. The solvent was removed by rotary evaporation to afford pure compound **2d** (1.6 g, 50% yield). MS [M+H]⁺: calcd. 285.1, found 285.1.

### (2) Preparation of compound 3d.

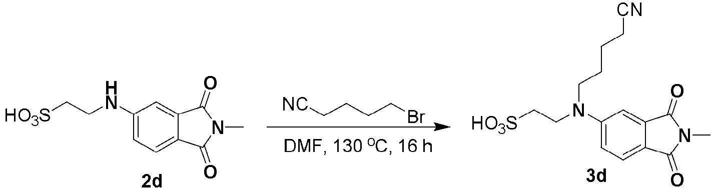

Compound **2d** (1.0 g, 3.52 mmol), DMF (10 mL), and pentabromovaleronitrile (5.7 g, 35.2 mmol) were added to a sealed tube, the mixture was stirred at 130 °C for 16 hours. The reaction was monitored by LC-MS, until starting material was basically converted into the target product. After the reaction, the crude liquid was purified by reversed-phase preparation. The solution of compound **3d** was obtained by purification with a reversed-phase C18 column (CH3CN/H2O, 0.1% FA), the solvent was then removed by rotary evaporation to obtain pure compound **3d** (1.0 g, 78% yield). MS [M+H]⁺: calcd. 366.1, found 366.1_{∘}

### (3) Preparation of compound 4d.

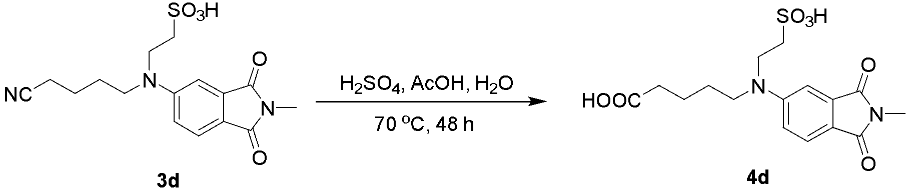

First, H₂O (1 mL), glacial acetic acid (1 mL), and concentrated sulfuric acid (2 mL) were added sequentially to a sealed tube. After mixing uniformly, compound **3d** (500 mg, 1.37 mmol) was added, and the mixture was heated and stirred at 70 °C for 48 hours. Upon completion of the reaction, the mixture was cooled to room temperature, diluted with H₂O (15 mL), and then purified by reversed-phase chromatography. Purification using a reversed-phase C18 column with eluent (CH₃CN/H₂O, 0.1% TFA) afforded pure compound **4d** (400 mg, 76% yield). MS [M+H]⁺: calcd. 385.1, found 385.1.

### (4) Preparation of compound LM-D.

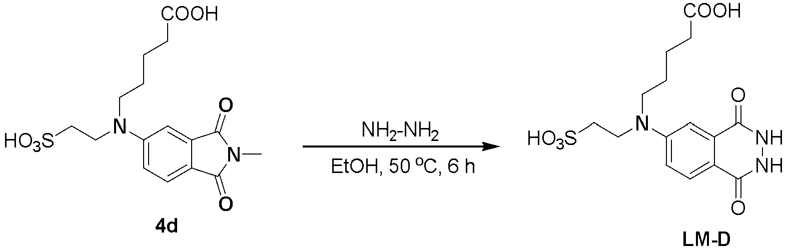

To a Schlenk tube, compound **4d** (200 mg, 0.52 mmol), ethanol (2 mL), and 85% hydrazine hydrate (520 mg, 8.83 mmol) were added. The mixture was heated and stirred at 50 °C for 6 hours. After the reaction was completed, the solvent and excess hydrazine hydrate were removed by rotary evaporation under reduced pressure to obtain a crude product. 3 N hydrochloric acid was added dropwise to the crude product until it became acidic (pH = 1-2), stirring should be maintained during the neutralization process, and the pH endpoint should remain stable for more than 10 minutes. Water and acetonitrile were added to dissolve the sample, and the crude solution was directly used for reversed-phase purification with a C18 column (CH₃CN/H₂O, 0.1% FA) afforded pure compound **LM-D** (150 mg, 75% yield).1H NMR (400 MHz, DMSO) δ 7.91 (s, 1H), 7.83 (d, J = 9.0 Hz, 1H), 7.15 (dd, J = 9.1, 2.8 Hz, 1H), 7.05 (d, J = 2.8 Hz, 1H), 3.71 - 3.62 (m, 2H), 3.41 (t, J = 6.9 Hz, 2H), 2.73 (t, J = 7.9 Hz, 2H), 2.18 (t, J = 6.8 Hz, 2H), 1.55 (dq, J = 9.8, 6.1, 5.2 Hz, 4H). MS [M+H]⁺: calcd. 386.1, found 386.2.

### Carboxyl activation of LM-D.

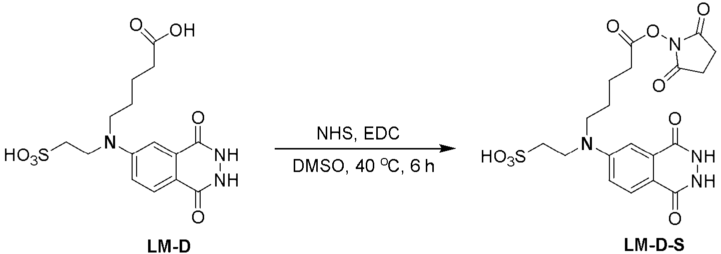

Compound **LM-D** (100 mg, 0.26 mmol) and NHS (119 mg, 1.03 mmol) were added to a Schlenk tube, followed by the addition of DMSO (5 mL). The mixture was firstly stirred at 40 °C for 5 minutes under the protection of argon. Then, the entire batch of EDC (149 mg, 0.78 mmol) was quickly added to the reaction solution, and the mixture was stirred at 40 °C for 6 hours. The reaction was monitored by LC-MS until the conversion of target product exceeded 70%, and then the reaction was stopped. After the reaction, the DMSO solution of the chemiluminescent label (the activated ester **LM-D-S)** was obtained, which could be directly used for protein labeling. MS [M+H]⁺: calcd. 483.1, found 483.1.

### Example 5

The synthetic route of **LM-E** is shown as follows:

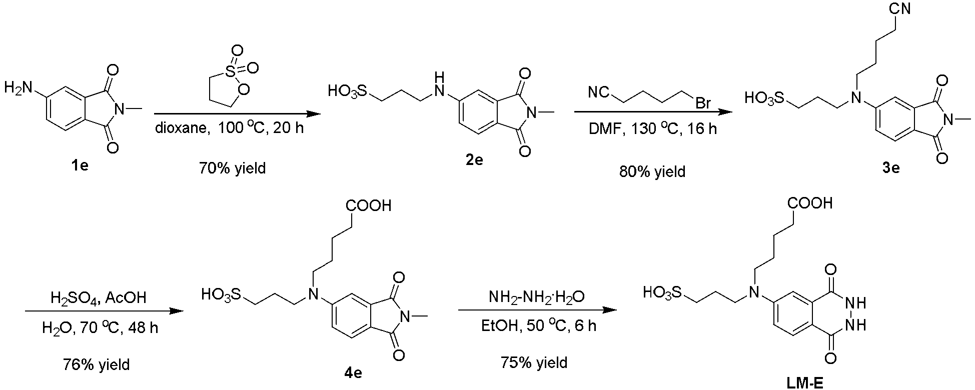

### (1) Preparation of compound 2e.

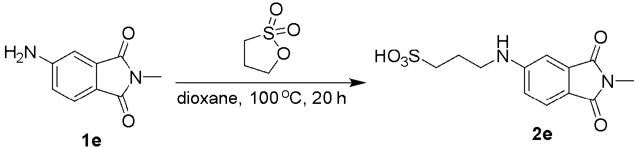

To a Schlenk tube, compound **1e** (3 g, 17.03 mmol), 1,3-propane sultone (20.8 g, 170.3 mmol) and dioxane (55 mL) were added. The mixture was heated at 100 °C for 20 hours under the protection of argon. The reaction solution was monitored by LC-MS and stopped when conversion of product exceeded 70%. The reaction solution was filtered while hot (filtered immediately after taking out from the oil bath), solid was colledcted and rinsed repeatedly with 80 °C dioxane solution three times. The compound **2e** (3.2 g, 70% yield) was obtained after drying with rotary evaporation, which could be directly used in the next reaction. MS [M+H]⁺: calcd. 269.09, found 299.0.

### (2) Preparation of compound 3e.

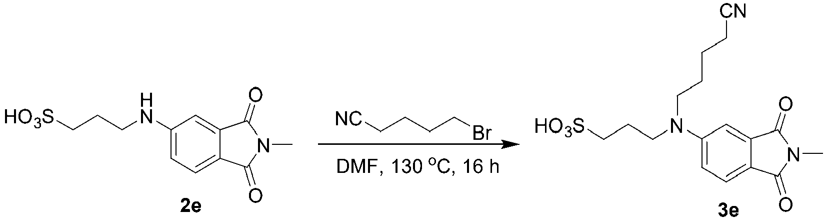

Compound **2e** (800 mg, 2.98 mmol), DMF (8 mL), and pentabromovaleronitrile (4.35 g, 26.85 mmol) were added to a sealed tube, the mixture was stirred at 130 °C for 16 hours. The reaction was monitored with LC-MS until the starting material was mostly converted to target product. After the reaction, the crude solution was purified by reversed-phase preparative chromatography on a C18 column using an eluent system of CH₃CN/H₂O (containing 0.1% FA), yielding a solution of compound **3e.** The acetonitrile was removed by rotary evaporation followed by lyophilization to obtain pure compound **3e** (904 mg, 80% yield). MS [M+H]⁺: calcd. 380.12, found 380.1.

### (3) Preparation of compound 4e.

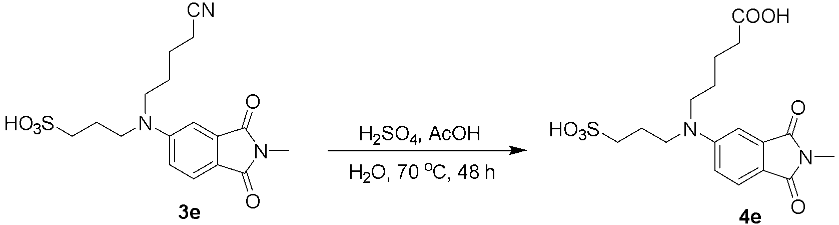

First, H₂O (4 mL), glacial acetic acid (4 mL), and concentrated sulfuric acid (8 mL) were added sequentially to a sealed tube. After thorough mixing, compound **3e** (800 mg, 2.1 mmol) was added, and the mixture was heated with stirring at 70 °C for 48 hours. Upon completion of the reaction, the mixture was cooled to room temperature, diluted with acetonitrile, and then subjected to reversed-phase purification using a C18 column with an eluent system of CH₃CN/H₂O (0.1% TFA), yielding pure compound **4e** (635 mg, 76% yield). MS [M+H]⁺: calcd. 399.11, found 399.1.

### (4) Preparation of compound LM-E.

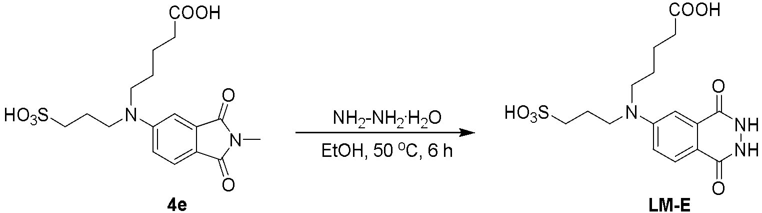

To a Schlenk tube, compound **4e** (700 mg, 1.76 mmol), ethanol (7 mL), and hydrazine hydrate (1.76 g, 35.2 mmol) were added. The mixture was heated with stirring at 50 °C for 6 hours. Upon completion, the solvent and excess hydrazine hydrate were removed by rotary evaporation to afford a crude product. 3 N hydrochloric acid was added dropwise to the crude product until acidic (pH = 1-2), stirring should be maintained during neutralization, and the pH endpoint should remain stable for at least 10 minutes. Water and acetonitrile were added to dissolve the sample, and the crude solution was directly subjected to reversed-phase purification on a C18 column with an eluent system of CH₃CN/H₂O (0.1% FA), yielding pure compound **LM-E** (527 mg, 75% yield). ¹H NMR (400 MHz, DMSO) δ 7.95 (s, 1H), 7.81 (d, J = 9.5 Hz, 1H), 7.22 (dd, J = 9.1, 2.8 Hz, 1H), 7.01 (s, 1H), 3.50 (t, J = 7.6 Hz, 2H), 3.42 (t, J = 6.7 Hz, 2H), 2.47 (t, J = 7.3 Hz, 2H), 2.31 - 2.22 (m, 2H), 1.84 (p, J = 7.5 Hz, 2H), 1.56 (p, J = 3.6 Hz, 4H). MS [M+H]⁺: calcd. 400.11, found 400.0.

**The activation procedure was the same as that** for **LM-D.**

### Example 6

The synthetic route of **LM-F** is shown as follows:

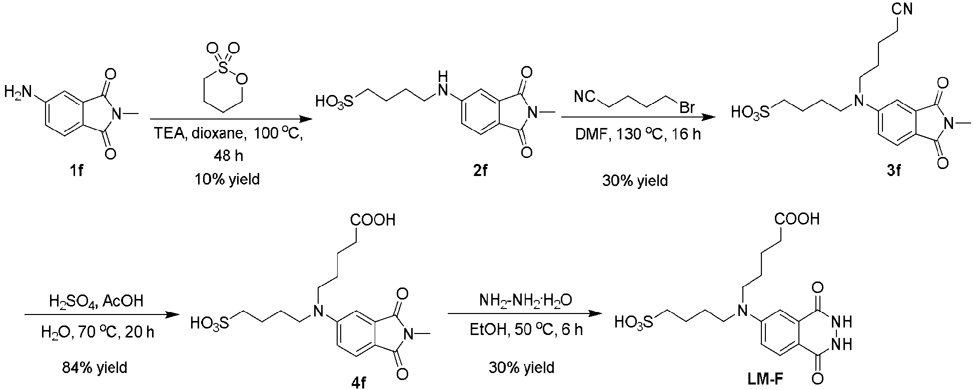

### (1) Preparation of compound 2f.

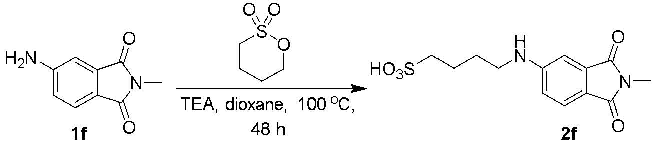

To a Schlenk tube, compound **1f** (3 g, 17.03 mmol), 1,4-butane sultone (23.2 g, 170.3 mmol), triethylamine (5.16 g, 51.09 mmol) and dioxane (60 mL) were added. Under argon protection, the mixture was heated at 100 °C for 48 hours. The reaction solution was monitored by LC-MS, showing a conversion of approximately 10%. After the reaction, the crude solution was purified by reversed-phase preparative chromatography on a C18 column with an eluent system of CH₃CN/H₂O (0.1% formic acid), yielding a solution of compound **2f.** The acetonitrile was removed by rotary evaporation followed by lyophilization to obtain pure compound **2f** (531 mg, 10% yield). MS [M+H]⁺: calcd. 313.08, found 313.0.

### (2) Preparation of compound 3f.

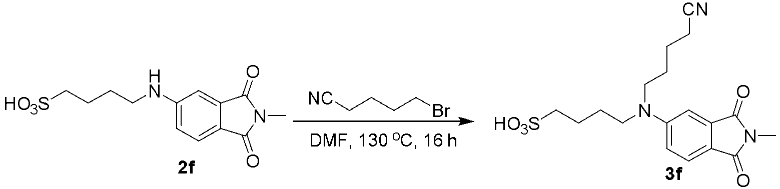

Compound **2f** (460 mg, 1.47 mmol), DMF (4 mL), and pentabromovaleronitrile (2.38 g, 14.7 mmol) were added to a sealed tube, and the mixture was stirred at 130 °C for 16 hours. The reaction was monitored with LC-MS until starting material was mostly converted to the target product. After completion of the reaction, the crude solution was purified by reversed-phase preparative chromatography using a C18 column with an eluent system of CH₃CN/H₂O (0.1% FA), yielding a solution of compound **3f.** The acetonitrile was removed by rotary evaporation followed by lyophilization to obtain pure compound **3f** (173 mg, 30% yield). MS [M+H]⁺: calcd. 394.14, found 394.0.

### (3) Preparation of compound 4f.

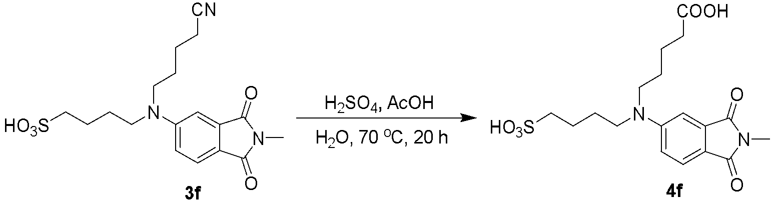

First, H₂O (1.5 mL), glacial acetic acid (1.5 mL), and concentrated sulfuric acid (3 mL) were added sequentially to a sealed tube. After thorough mixing, compound **3f** (160 mg, 0.4 mmol) was added, and the mixture was heated with stirring at 70 °C for 20 hours. Upon completion of the reaction, the mixture was cooled to room temperature and diluted with acetonitrile (15 mL), then subjected to reversed-phase purification. Purification using a C18 column with an eluent system of CH₃CN/H₂O (0.1% TFA) afforded pure compound **4f** (138 mg, 84% yield). MS [M+H]⁺: calcd. 413.13, found 413.1.

### (4) Preparation of compound LM-F.

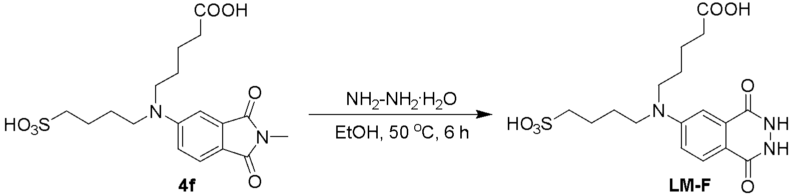

To a Schlenk tube, compound **4f** (140 mg, 0.34 mmol), ethanol (7 mL), and hydrazine hydrate (340 mg, 6.8 mmol) were added. The mixture was heated with stirring at 50 °C for 6 hours. Upon completion, the solvent and excess hydrazine hydrate were removed by rotary evaporation under reduced pressure to afford a crude product. 3 N hydrochloric acid was added dropwise to the crude product until acidic (pH = 1-2), stirring should be maintained during neutralization, and the pH endpoint should remain stable for at least 10 minutes. Water and acetonitrile were added to dissolve the sample, and the crude solution was directly subjected to reversed-phase purification on a C18 column with an eluent system of CH₃CN/H₂O (0.1% FA), yielding pure compound **LM-F** (42 mg, 30% yield). ¹H NMR (400 MHz, DMSO) δ 7.82 (d, *J* = 9.0 Hz, 1H), 7.19 (dd, *J* = 9.0, 2.7 Hz, 1H), 7.02 (d, *J =* 2.6 Hz, 1H), 3.40 (q, *J* = 6.3 Hz, 4H), 2.56 (t, *J* = 7.3 Hz, 2H), 2.28 - 2.24 (m, 2H), 1.69 - 1.62 (m, 4H), 1.56 (p, *J* = 3.8 Hz, 4H). MS [M+H]⁺: calcd. 414.13, found 414.0.

**The activation procedure was the same as that for LM-D.**

### Example 7

The synthetic route of **LM-G** is shown as follows:

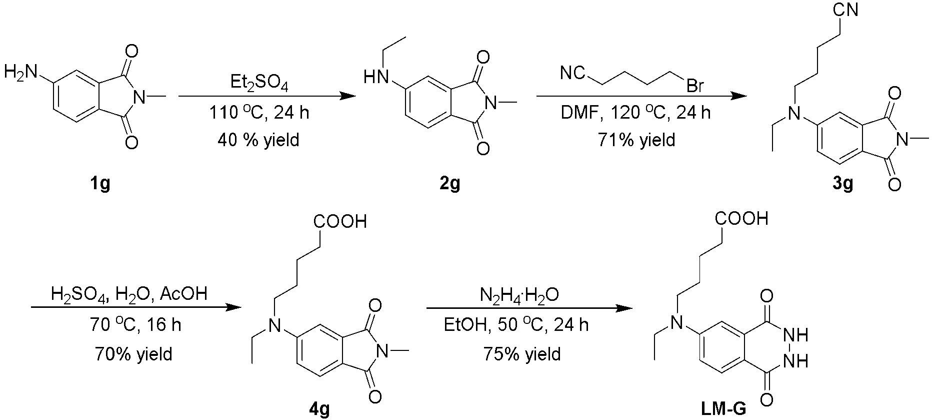

### (1) Preparation of compound 2g.

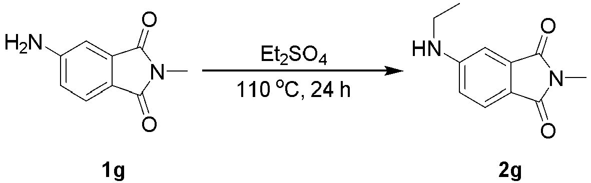

To a three-necked flask, compound **1g** (5.0 g, 28.4 mmol) and diethyl sulfate (50 mL, 379 mmol) were added. Under argon protection, the mixture was heated at 110 °C for 24 hours. The reaction was monitored by TLC (petroleum ether : ethyl acetate = 10:1) until the complete conversion of the starting material. After completion, the system was cooled to room temperature, H₂O (50 mL) was added and kept stirring for 5 minutes. The mixture was then extracted with dichloromethane (200 mL). The organic phase was collected and dried, then the solvent was removed to obtain crude compound **2g,** which was further purified by column chromatography to give pure compound **2g** (2.32 g, 40% yield). MS [M+H]⁺: calcd. 205.1, found 205.5.

### (2) Preparation of compound 3g.

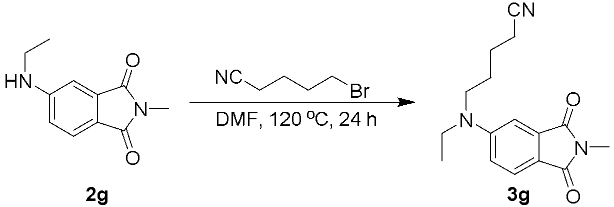

Compound **2g** (2.32 g, 11.4 mmol) was dissolved in DMF (50 mL), and then pentabromovaleronitrile (3.7 g, 22.8 mmol) was added. The mixture was heated at 120 °C for 24 hours under argon protection. After the reaction was completed, extraction was performed with ethyl acetate. The organic phase was dried, and the solvent was removed under reduced pressure to obtain crude compound **3g.** Further purification by column chromatography afforded pure compound **3a** (2.3 g, 71% yield). MS [M+H]⁺: calcd. 286.2, found 286.4.

### (3) Preparation of compound 4g.

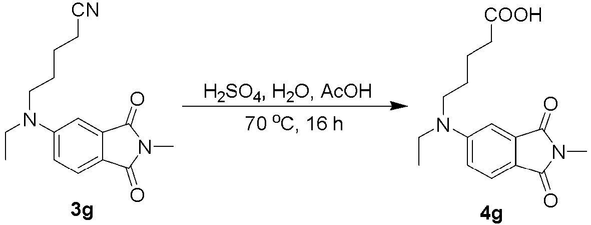

To a reaction flask, H₂O (2 mL), glacial acetic acid (2 mL), and concentrated sulfuric acid (2 mL) were added sequentially. After thorough mixing, compound **3g** (2.2 g, 7.71 mmol) was added. The mixture was heated and stirred at 70 °C for 16 hours. Upon completion of the reaction, the mixture was cooled to room temperature and diluted with H₂O (10 mL). Solid sodium bicarbonate was then added slowly until no more bubbles were generated. DMF (10 mL) was added to clarify the solution, and this clear solution was directly used for reverse-phase purification. Purification by reverse-phase chromatography on a C18 column with an eluent system of CH₃CN/H₂O (0.1% FA), yielding pure compound **4g** (1.64 g, 70% yield). MS [M+H]⁺: calcd. 305.1, found 305.5.

### (4) Preparation of compound LM-G.

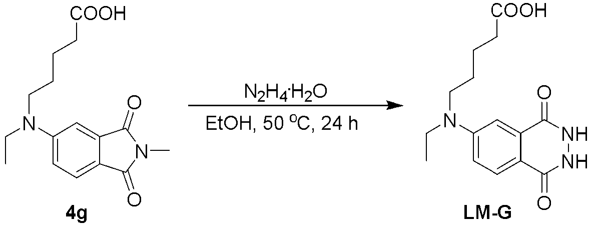

Compound **4g** (5.5 g, 18.1 mmol) was dissolved in ethanol (55 mL), and hydrazine hydrate (18.12 g, 362 mmol) was added. The mixture was heated and stirred at 50 °C for 24 hours. After the reaction was completed, 1 N hydrochloric acid was added dropwise until the solution became weakly acidic. The solvent and excess hydrazine hydrate were removed by rotary evaporation under reduced pressure. DMF (100 mL) was added to dissolve the sample, resulting in a clear solution, which was directly used for reverse-phase purification. Purification by reverse-phase chromatography on a C18 column (CH₃CN/H₂O with 0.1% TFA) afforded pure compound **LM-G** (4.1 g, 75% yield). ¹H NMR (400 MHz, DMSO) δ 11.53 (s, 3H), 7.82 (d, *J* = 8.9 Hz, 1H), 7.17 (dd, *J* = 9.0, 2.7 Hz, 1H), 7.02 (s, 1H), 3.51 - 3.44 (m, 2H), 3.40 (t, *J =* 5.1 Hz, 2H), 2.31 - 2.23 (m, 2H), 1.57 (p, *J* = 3.6 Hz, 4H), 1.13 (t, *J* = 7.0 Hz, 3H). MS [M+H]⁺: calcd. 306.1, found 306.5.

### Carboxyl activation of LM-G.

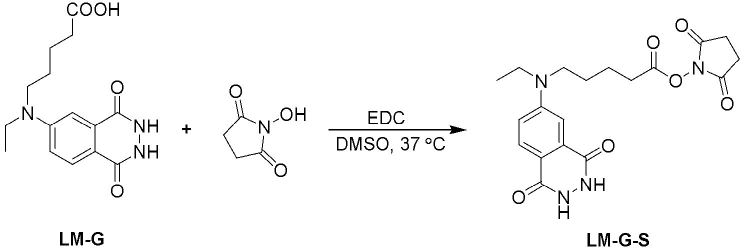

Compound **LM-G** (100 mg, 0.328 mmol) was dissolved in DMSO (5 mL), followed by the addition of EDC (125.6 mg, 0.655 mmol). Under argon protection, the mixture was stirred at room temperature for 20 minutes, then NHS (75.4 mg, 0.655 mmol) was added, and the mixture was stirred at 40 °C for further 12 hours. After the reaction was completed, the crude mixture was purified by reverse-phase chromatography on a C18 column (CH₃CN/H₂O with 0.1% TFA) and then lyophilized to obtain compound **LM-G-S** (58 mg, 44% yield). MS [M+H]⁺: calcd. 403.2, found 403.2.

### Example 8

The synthetic route of **LM-H** is shown as follows:

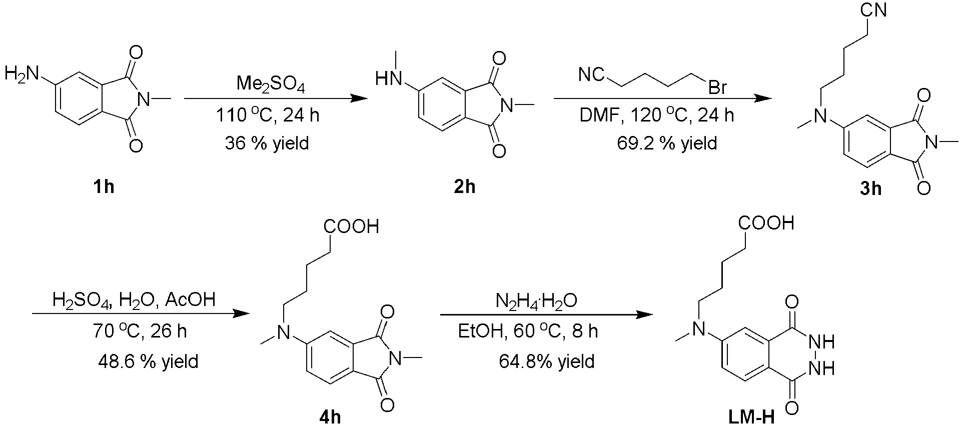

### (1) Preparation of compound 2h.

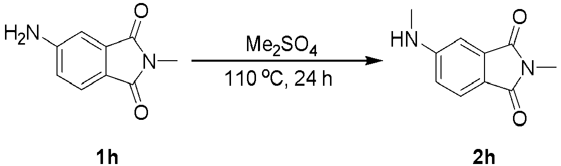

To a three-necked flask, compound 1h (5.0 g, 28.4 mmol) and dimethyl sulfate (30 mL, 317 mmol) were added. Under argon protection, the mixture was heated at 110 °C for 24 hours. The reaction was monitored by TLC (petroleum ether : ethyl acetate = 10:1) until the complete conversion of the starting material. After completion, the system was cooled to room temperature and H₂O (50 mL) was added, the mixture was extracted with dichloromethane. The organic phase was collected and dried, solvent was removed to obtain crude compound **2h,** which was further purified by column chromatography to give pure compound **2h** (1.94 g, 36% yield). MS [M+H]⁺: calcd. 191.07, found 191.1.

### (2) Preparation of compound 3h.

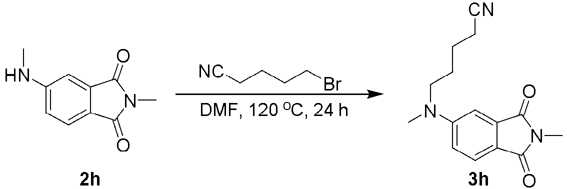

Compound **2h** (280 mg, 1.472 mmol) was dissolved in DMF (5 mL), and then pentabromovaleronitrile (1.2 g, 7.41 mmol) was added. The mixture was heated at 120 °C for 24 hours under argon protection. The reaction was monitored by LC-MS until starting material was basically converted into the target product. After the reaction was completed, the crude reaction solution was directly purified by silica gel column chromatography to obtain pure compound **3h** (278 mg, 69.2% yield). MS [M+H]⁺: calcd. 272.13, found 273.0. This step was repeated twice.

### (3) Preparation of compound 4h.

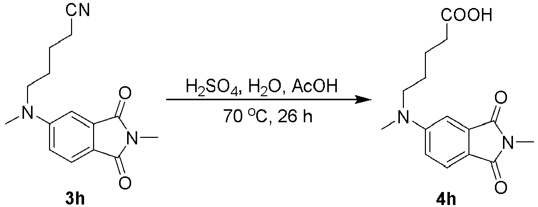

To a reaction flask were added sequentially H₂O (2 mL), glacial acetic acid (2 mL), and concentrated sulfuric acid (4 mL). After thorough mixing, compound **3h** (500 mg, 1.843 mmol) was added, and the mixture was heated with stirring at 70 °C for 26 hours. Upon completion of the reaction, the mixture was cooled to room temperature and diluted by adding H₂O (10 mL). This solution was directly used for reversed-phase purification on a C18 column using CH₃CN/H₂O (with 0.1% TFA) as eluent, yielding pure compound **4h** (261 mg, 48.6% yield). MS [M+H]⁺: calcd. 291.13, found 291.0.

### (4) Preparation of compound LM-H.

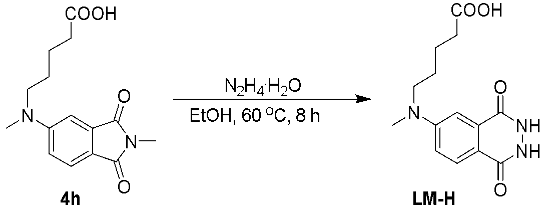

Compound **4h** (200 mg, 0.689 mmol) was dissolved in ethanol (5 mL), then hydrazine hydrate (689 mg) was added. The mixture was heated and stirred at 60 °C for 8 hours. The reaction was monitored by LC-MS until the starting material was basically converted into target product. After the reaction was completed, 1 N hydrochloric acid was added dropwise until the solution was weakly acidic. The solvent and excess hydrazine hydrate were removed by rotary evaporation under reduced pressure. DMF (10 mL) was added to dissolve the sample, resulting in a clear solution, which was directly used for reversed-phase purification. Pure compound **LM-H** (131 mg, 64.8% yield) was obtained after purification by reversed-phase C18 column chromatography (CH₃CN/H₂O with 0.1% TFA). ¹H NMR (400 MHz, DMSO) δ 11.41 (s, 3H), 7.84 (d, *J* = 8.9 Hz, 1H), 7.22 (dd, *J* = 9.0, 2.7 Hz, 1H), 7.02 (d, *J* = 2.6 Hz, 1H), 3.51 - 3.44 (m, 2H), 3.02 (s, 3H), 2.28 - 2.23 (m, 2H), 1.61 - 1.49 (m, 4H). MS [M+H]⁺: calcd. 292.12, found 292.0.

### Carboxyl activation of LM-H.

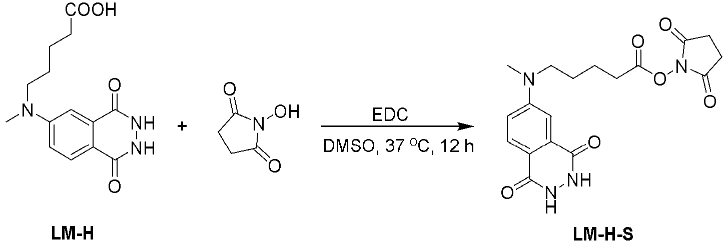

Compound **LM-H** (100 mg, 0.3435 mmol) was dissolved in DMSO (5 mL). NHS (158 mg, 1.375 mmol) was added, and the mixture was stirred at room temperature under argon protection for 5 minutes. Then EDC (200 mg, 1.05 mmol) was added, and the mixture was stirred at 37 °C for further 12 hours. After the reaction was completed, purification was performed using a reversed-phase C18 column (CH₃CN/H₂O with 0.1% TFA) to obtain a solution of ester product, which was further freeze-dried to get pure compound **LM-H-S** (68 mg, 51% yield). MS [M+H]⁺: calcd. 389.14, found 389.0.

### Example 9

The synthetic route of **LM-I** is shown as follows:

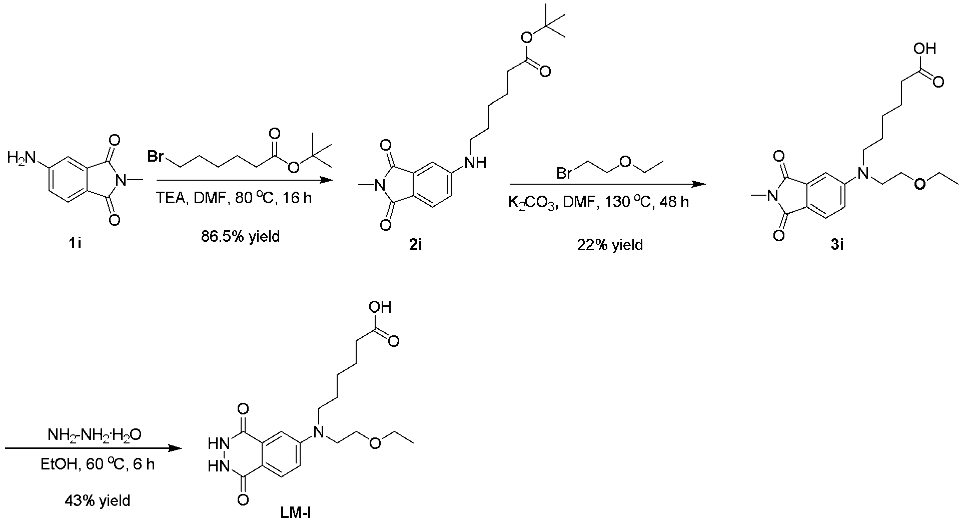

### (1) Preparation of compound 2i.

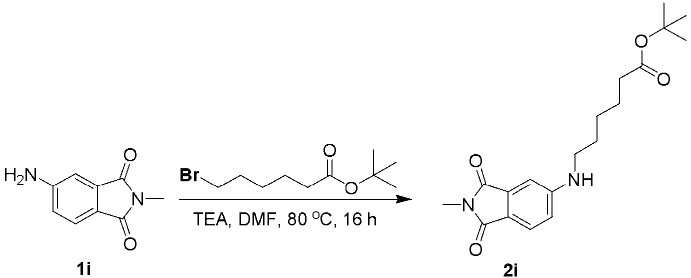

To a Schlenk tube were added compound **1i** (3 g, 17.03 mmol), tert-butyl 6-bromohexanoate (21.4 g, 85.15 mmol), DMF (45 mL) and triethylamine (8.6 g, 85.15 mmol). Under argon protection, the mixture was heated at 80 °C for 16 hours. The reaction solution was monitored by LC-MS until starting material was basically converted into the product. After the reaction was completed, the crude solution was roughly purified by reversed-phase preparation using a C18 column (CH₃CN/H₂O with 0.1% FA) to obtain a solution of compound **2i.** Acetonitrile was removed by rotary evaporation, and the residue was freeze-dried to give crude compound **2i** (5.1 g, 86.5% yield), which could be directly used in the next step. MS [M-(t-BuO⁻)]⁺: calcd. 273.12, found 272.0.

### (2) Preparation of compound 3i.

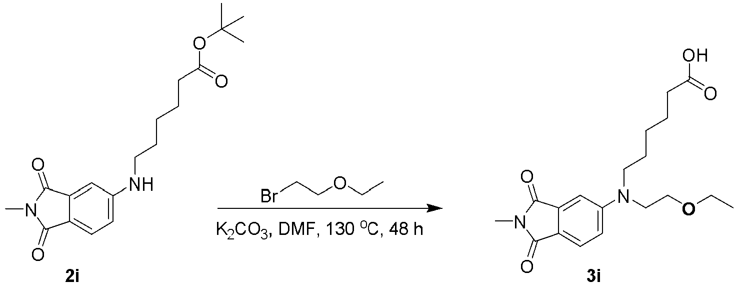

Compound **2i** (1 g, 2.89 mmol), potassium carbonate (1.2 g, 8.67 mmol), DMF (10 mL) and 2-bromoethyl ethyl ether (4.42 g, 28.9 mmol) were added into a sealed tube. The mixture was stirred at 130 °C for 48 hours. The reaction was monitored by LC-MS, showing that the conversion of the starting material was about 30%. The reaction was stopped, the crude mixture was roughly purified by reversed-phase preparation using a C18 column (CH₃CN/H₂O with 0.1% FA) to obtain a solution of compound **3i.** Acetonitrile was removed by rotary evaporation, the remaining aqueous phase was then extracted with dichloromethane. The organic solvent was evaporated to dryness to give a crude product of compound 3i. The crude **3i** was further purified by silica gel column chromatography, yielding the pure **3i** (230.3 mg, 22% yield). MS [M+H]⁺: calcd. 363.18, found 363.0.

### (3) Preparation of compound LM-I.

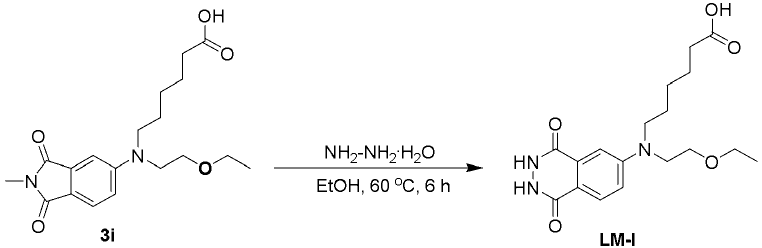

To a Schlenk tube, compound **3i** (230 mg, 0.635 mmol), ethanol (10 mL) and hydrazine hydrate (635 mg, 12.7 mmol) were added. The mixture was heated and stirred at 60 °C for 6 hours. After the reaction was completed, the solvent and excess hydrazine hydrate were removed by rotary evaporation under reduced pressure to obtain a crude product. 3 N hydrochloric acid was added dropwise to the crude product until it became acidic (pH=1-2), stirring should be maintained during the neutralization process, and the pH endpoint should remain stable for more than 10 minutes. Water and acetonitrile were added to dissolve the sample, and the crude sample solution was directly used for reversed-phase purification. Pure compound **LM-I** (99.2 mg, 43% yield) was obtained after purification on a C18 column chromatography (CH₃CN/H₂O with 0.1% formic acid). ¹H NMR (400 MHz, DMSO) δ 11.40 (s, 3H), 7.82 (d, *J* = 9.0 Hz, 1H), 7.19 (dd, *J* = 9.0, 2.7 Hz, 1H), 7.05 (s, 1H), 3.63 - 3.52 (m, 4H), 3.44 (q, *J* = 7.0 Hz, 4H), 2.22 (t, *J* = 7.3 Hz, 2H), 1.55 (dq, J = 15.3, 7.7 Hz, 4H), 1.37 - 1.28 (m, 2H), 1.09 (t, *J* = 7.0 Hz, 3H). MS [M+H]⁺: calcd. 364.18, found 364.0.

### Carboxyl activation of LM-I.

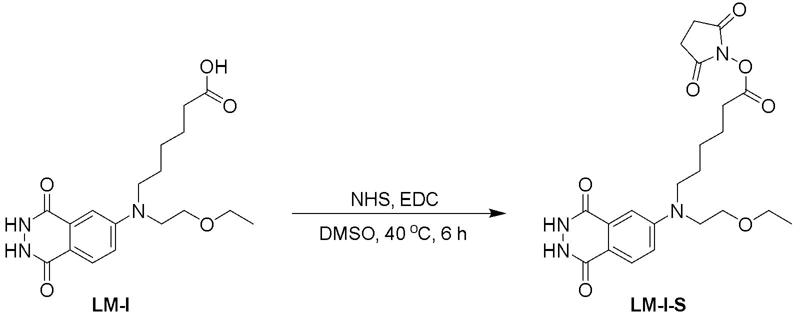

**LM-I** (100 mg, 0.275 mmol) and NHS (126.7 mg, 1.10 mmol) were added into a Schlenk tube, followed by the addition of DMSO (4 mL). The mixture was stirred at 40 °C for 5 minutes with argon protection, then the entire batch of EDC (158.2 mg, 0.825 mmol) was quickly added to the reaction solution, and the mixture was stirred at 40 °C for further 6 hours. The reaction was monitored by LC-MS until starting material was basically converted into the target product. After the reaction was completed, the crude mixture was directly used for reversed-phase purification. It was purified by reversed-phase C18 column chromatography (CH₃CN/H₂O with 0.1% FA) to obtain the eluent containing the target product, which was freeze-dried to get the pure **LM-I-S** (60.7 mg, 48% yield). MS [M+H]⁺: calcd. 461.20, found 461.2.

### Example 10

The synthetic route of **LM-J** is shown as follows:

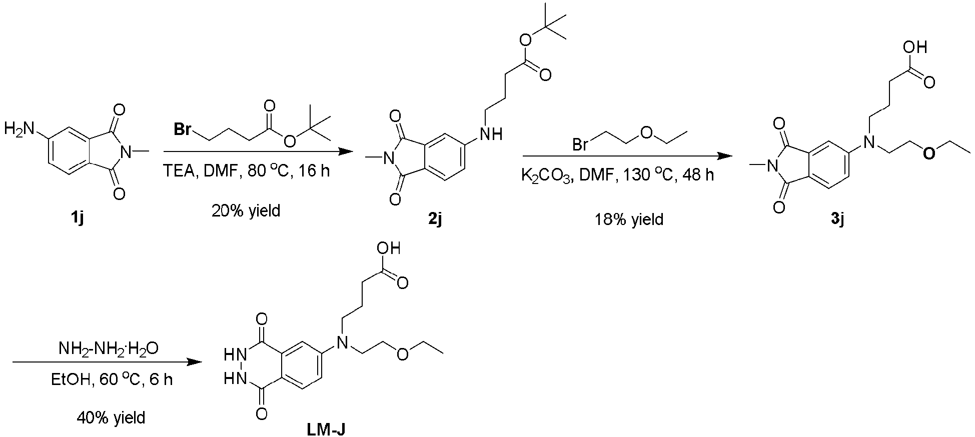

### (1) Preparation of compound 2j.

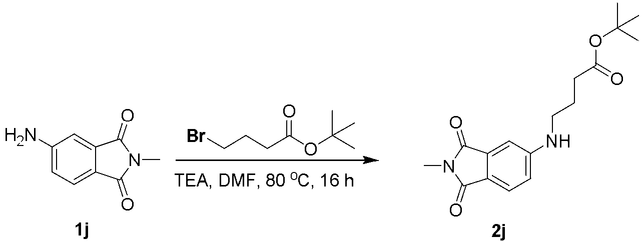

To a Schlenk tube, compound **1j** (3 g, 17.03 mmol), tert-butyl 4-bromobutyrate (19 g, 85.15 mmol), DMF (45 mL) and triethylamine (8.6 g, 85.15 mmol) were added. Under argon protection, the mixture was heated at 80 °C for 16 hours. The reaction was monitored by LC-MS, showing a conversion of approximately 25%. The reaction was stopped, the crude solution was purified by reversed-phase preparation using a C18 column (CH₃CN/H₂O with 0.1% FA) to obtain a solution of compound 2j. After rotary evaporation and freeze-drying, pure compound **2j** (1084 mg, 20% yield) was obtained. MS [M-(t-BuO⁻)]⁺: calcd. 245.09, found 245.0.

### (2) Preparation of compound 3j.

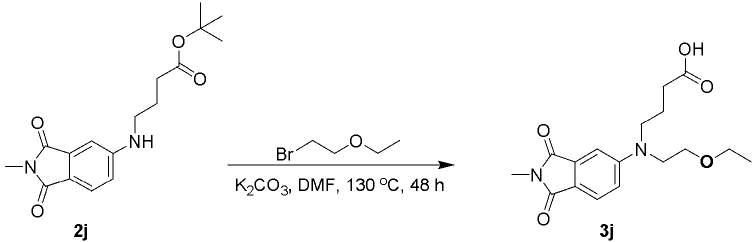

Compound **2j** (450 mg, 1.41 mmol), potassium carbonate (586 mg, 4.24 mmol), DMF (5 mL) and 2-bromoethyl ethyl ether (2.16 g, 14.1 mmol) were added into a sealed tube. The mixture was stirred at 130 °C for 48 hours. The reaction was monitored by LC-MS which showed a conversion of the starting material was approximately 20%. The reaction was stopped, the crude solution was roughly purified by reversed-phase preparative chromatography using a C18 column (CH₃CN/H₂O with 0.1% formic acid) to obtain a solution of compound **3j.** Acetonitrile was removed by rotary evaporation, and the remaining aqueous phase was extracted with dichloromethane. The organic solvent was evaporated to dryness to give a crude product of compound **3j.** The crude **3j** was further purified by silica gel column chromatography, yielding pure **3j** (84.8 mg, 18% yield). MS [M+H]⁺: calcd. 335.15, found 335.0.

### (3) Preparation of LM-J.

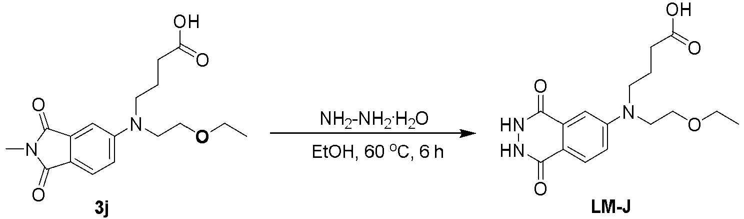

To a Schlenk tube, compound **3j** (85 mg, 0.254 mmol), ethanol (4 mL) and hydrazine hydrate (101.6 mg, 5.08 mmol) were added. The mixture was heated and stirred at 60 °C for 6 hours. After the reaction was completed, the solvent and excess hydrazine hydrate were removed by rotary evaporation under reduced pressure to obtain a crude product. 3 N hydrochloric acid was added dropwise to the crude product until it became acidic (pH=1-2), stirring should be maintained during the neutralization process, and the pH endpoint should remain stable for more than 10 minutes. Water and acetonitrile were added to dissolve the sample, and the crude mixture was directly used for reversed-phase purification. Pure compound **LM-J** (34 mg, 40% yield) was obtained after purification on a C18 column chromatography (CH₃CN/H₂O with 0.1% FA). ¹H NMR (400 MHz, DMSO) δ 8.42 (s, 1H), 7.82 (d, *J =* 8.9 Hz, 1H), 7.27 (d, *J* = 9.0 Hz, 1H), 7.11 (s, 1H), 3.57 (dd, *J* = 12.6, 4.7 Hz, 4H), 3.44 (q, *J* = 7.0 Hz, 4H), 2.21 (t, *J* = 7.1 Hz, 2H), 1.75 (q, *J =* 7.5 Hz, 2H), 1.09 (t, *J =* 7.0 Hz, 3H). MS [M+H]⁺: calcd. 336.15, found 336.0.

**The activation step was the same as that for TM-I.**

### Example 11

The synthetic route of **LM-K** is shown as follows:

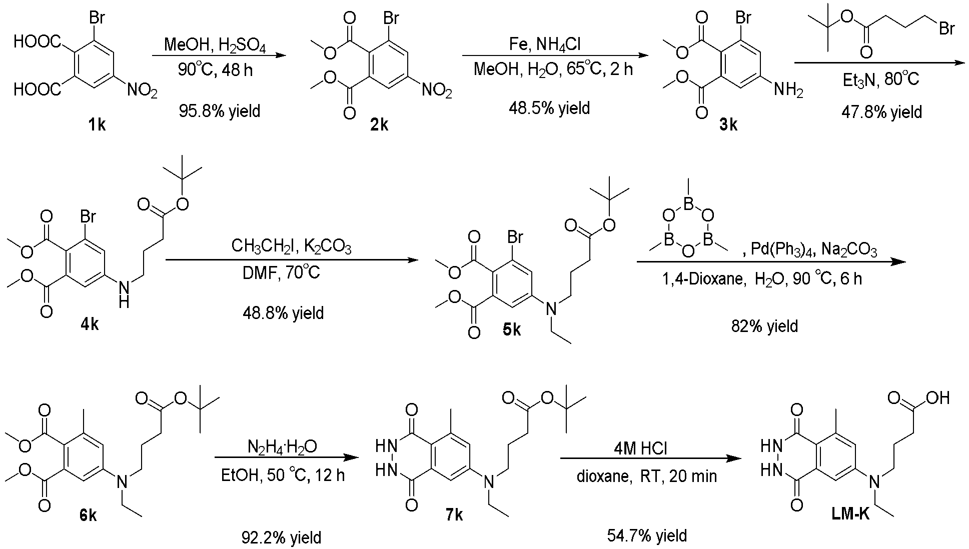

### (1) Preparation of compound 2k.

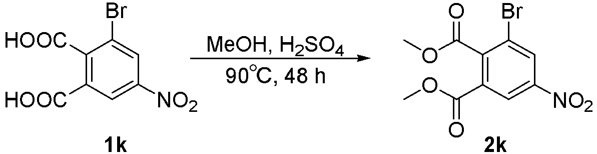

Compound 1k (2.51 g, 8.69 mmol) was added to a pressure tube and dissolved in 100 mL of methanol to form a clear solution. Concentrated sulfuric acid (100 mL) was then added under an ice-water bath, after which the pressure tube was hermetically sealed and resulting mixture was heated at 90°C for 48 hours. The reaction was monitored by TLC (petroleum ether : ethyl acetate = 8:1) until complete conversion of the starting material. Upon completion, the reaction was quenched by adding 1 L of ice water, followed by extraction with 1 L of ethyl acetate. The organic phase was collected, then dried and concentrated by rotary evaporation to afford crude compound **2k** (2.64 g, 95.8% yield).

### (2) Preparation of compound 3k.

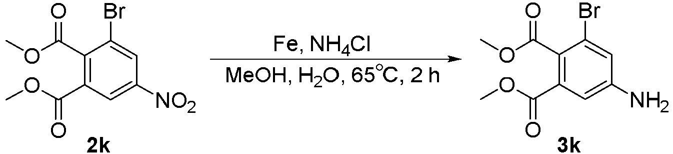

Compound **2k** (2.64 g, 8.33 mmol) was charged into a reaction flask and dissolved in 150 mL of methanol. Separately, ammonium chloride (2.2 g, 41.6 mmol) was dissolved in 50 mL of water, and this aqueous solution was added to the aforementioned methanolic solution. Finally, iron powder (4.66 g, 83.3 mmol) was introduced. The reaction was heated at 65°C for 2 hours under a nitrogen atmosphere, with progress monitored by TLC (petroleum ether:ethyl acetate = 2:1) until complete consumption of the starting material. The reaction mixture was filtered through Celite, and the collected filtrate was concentrated via rotary evaporation to give crude compound 3k. Purification of the crude product by silica gel column chromatography afforded pure compound **3k** (1.16 g, 48.5% yield).

### (3) Preparation of compound 4k.

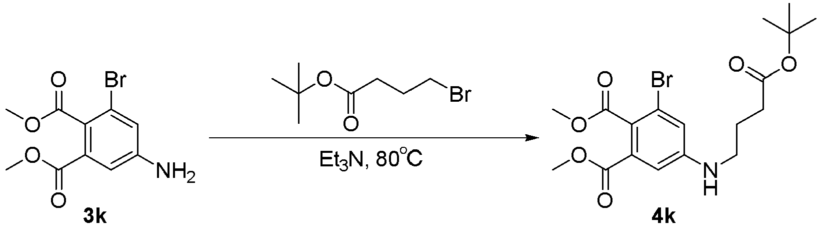

Compound **3k** (1.1 g, 3.83 mmol), tert-butyl 4-bromobutyrate (8.5 g, 38.3 mmol) and triethylamine (3.88 g, 38.3 mmol) were sequentially added to a pressure tube, which was then hermetically sealed. The reaction was heated at 80°C for 24 hours, with progress monitored by TLC (petroleum ether:ethyl acetate = 2:1) until most of the starting material was converted. Upon completion, the reaction mixture was extracted with ethyl acetate. The organic phase was dried and concentrated by rotary evaporation to obtain crude compound **4k.** Purification of the crude product by silica gel column chromatography afforded pure compound **4k** (0.56 g, 47.8% yield).

### (4) Preparation of compound 5k.

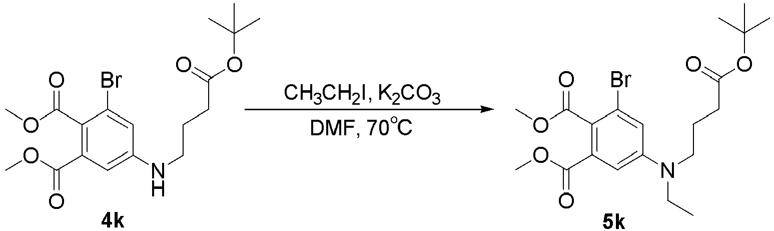

Compound **4k** (0.56 g, 1.3 mmol) was placed in a pressure tube and dissolved in 7 mL of DMF. lodoethane (6.08 g, 39 mmol) and potassium carbonate (0.54 g, 3.9 mmol) were then added, and the pressure tube was hermetically sealed. The reaction was heated at 70°C for 14 hours, with progress monitored by TLC (petroleum ether:ethyl acetate = 4:1) until most of the starting material had been converted. Upon completion, the reaction mixture was extracted with ethyl acetate. The organic phase was dried and concentrated by rotary evaporation to yield crude compound **5k.** Purification via silica gel column chromatography afforded pure compound **5k** (0.29 g, 48.8% yield). MS [M+H]⁺: calcd. 458.12, found 457.9.

### (5) Preparation of compound 6k.

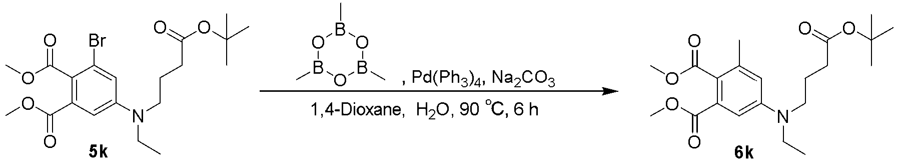

A Schlenk tube was charged with compound **5k** (0.15 g, 0.33 mmol), tetrakis(triphenylphosphine)palladium (38 mg, 0.033 mmol), sodium carbonate (277.7 mg, 2.62 mmol), dioxane (5 mL) and water (1 mL). Trimethylboroxine (383 µL, 1.31 mmol) was added under nitrogen protection. The reaction mixture was heated at 90°C for 6 hours, with progress monitored by LC-MS until complete conversion of the starting material. Upon completion, the reaction mixture was filtered through Celite, and the filtrate was concentrated by rotary evaporation to afford crude compound **6k.** The crude product was further purified by silica gel column chromatography (petroleum ether:ethyl acetate = 4:1) to give pure compound **6k** (106 mg, 82% yield). MS [M+H]⁺: calcd. 394.22, found 394.2.

### (6) Preparation of compound 7k.

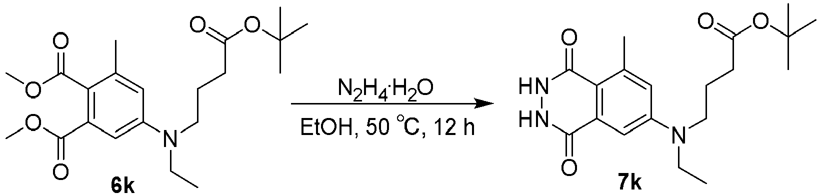

A Schlenk tube was charged with compound **6k** (106 mg, 0.27 mmol) and dissolved in 5 mL of ethanol, followed by the addition of 85% hydrazine hydrate (0.6 mL, 10 mmol). The reaction was heated at 50°C for 24 hours, with progress monitored by LC-MS until most of the starting material had been converted. The reaction mixture was concentrated by rotary evaporation, then 50 mL of water was added and the pH was adjusted to 2. Ethyl acetate (50 mL) was added for extraction. The organic phase was collected and concentrated by rotary evaporation to afford crude compound **7k** (90 mg, 92.2% yield). MS [M+H]⁺: calcd. 362.21, found 362.4.

### (7) Preparation of compound LM-K.

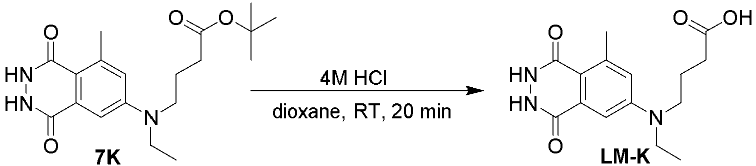

The entire amount of crude compound **7k** obtained from the previous step was taken and treated with 4 M HCl in dioxane (3 mL). The reaction was stirred at room temperature for 20 minutes, with progress monitored by LC-MS until most of the starting material had been converted. The reaction mixture was concentrated by rotary evaporation and purified by reverse-phase C18 column chromatography (CH₃CN/H₂O with 0.1% TFA) to afford pure compound **LM-K** (45 mg, 54.7% yield). ¹H NMR (400 MHz, DMSO) δ 6.97 (s, 1H), 6.91 (s, 1H), 2.71 (s, 3H), 2.31 (t, *J =* 7.1 Hz, 2H), 1.76 (q, *J* = 6.4, 5.3 Hz, 4H), 1.35 (s, 2H), 1.12 (t, *J =* 6.9 Hz, 3H). MS [M+H]⁺: calcd. 306.14, found 306.0.

**The activation procedure was the same as that for LM-B.**

### Example 12

The synthetic route of **LM-L** is shown as follows:

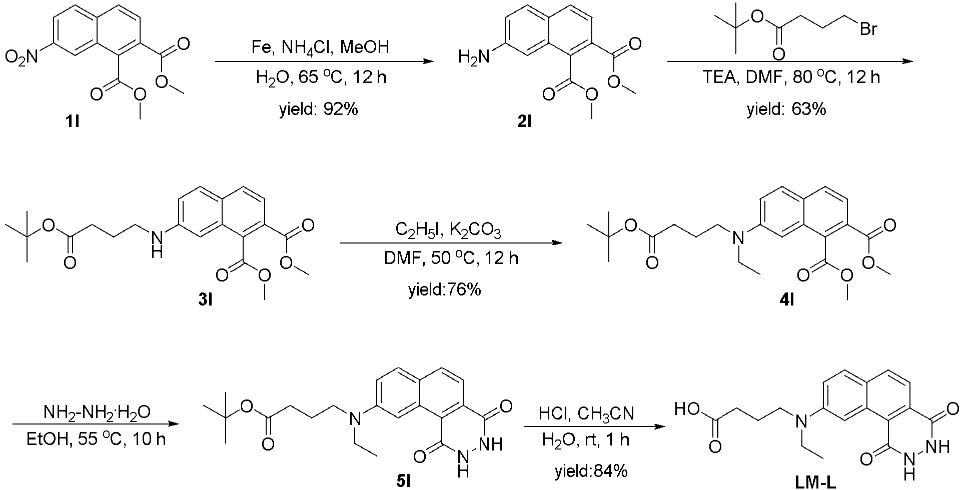

### (1) Preparation of compound 21.

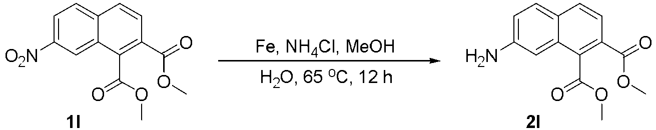

A round-bottom flask was charged with compound **1l** (3 g, 10.4 mmol) and ammonium chloride (2.8 g, 52.3 mmol), which were dissolved in methanol (90 mL) and water (30 mL). Subsequently, reduced iron powder (5.8 g, 103.6 mmol) was weighed and added to the above mixture. The reaction was conducted at 65°C for 12 hours under nitrogen protection. The reaction progress was monitored by TLC, and the reaction was stopped once the starting material was completely consumed. After completion, the reaction mixture was filtered through Celite, and the filtrate was collected. Most of the methanol was removed from the filtrate by rotary evaporation, followed by the addition of water (20 mL). The crude solution was then extracted with dichloromethane, the organic phase was collected, and the solvent was removed by rotary evaporation to obtain crude compound **2l** (2.5 g, 9.6 mmol, 92% yield). MS [M+H]⁺: calcd. 260.08, found 228.4.

### (2) Preparation of compound 3l.

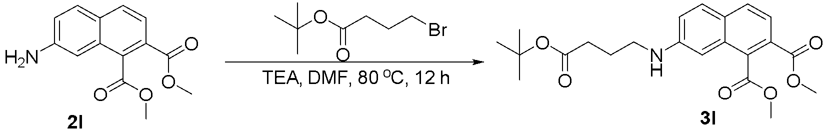

A sealed pressure tube was sequentially charged with compound **2l** (1 g, 3.9 mmol), DMF (15 mL), tert-butyl 4-bromobutyrate (3.6 mL, 20.3 mmol) and triethylamine (2.8 mL, 20.3 mmol). The tube was sealed and the reaction was conducted at 80°C for 12 hours. After completion, the reaction mixture was cooled to room temperature. The crude solution was directly injected as liquid sample and purified by reverse-phase C18 column chromatography (CH₃CN/H₂O with 0.1% TEA) to afford pure compound **3l** (1.0 g, 2.5 mmol, 63% yield).

### (3) Preparation of compound 41.

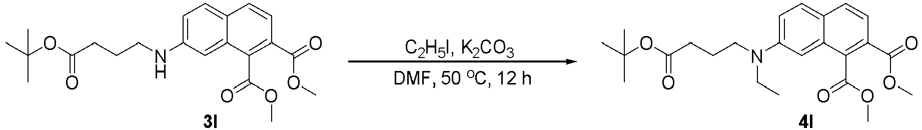

A sealed pressure tube was sequentially charged with compound **3l** (1.0 g, 2.5 mmol), DMF (15 mL), potassium carbonate (1 g, 7.2 mmol) and iodoethane (4 mL, 50 mmol). The tube was sealed and the reaction was carried out at 50°C for 12 hours. Upon completion, the reaction mixture was cooled to room temperature. The crude solution was directly injected as a liquid sample and purified by reverse-phase C18 column chromatography (CH₃CN/H₂O with 0.1% TEA) to afford pure compound **4l** (816.1 mg, 1.9 mmol, 76% yield). MS [M+H]⁺: calcd. 430.22, found 431.0.

### (4) Preparation of compound 51.

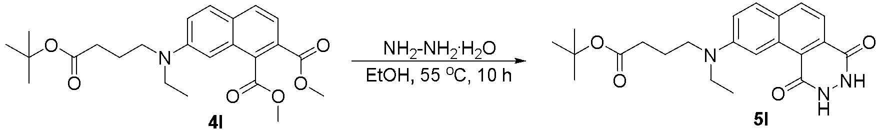

Compound **4l** (816.1 mg, 1.9 mmol) was dissolved in 8 mL of ethanol, and hydrazine hydrate (2.9 mL, 57 mmol) was added. The reaction was conducted at 50°C for 10 hours, with progress monitored by LC-MS until most of the starting material had been converted. Upon completion, ethanol was removed by rotary evaporation. The crude product was redissolved in 20 mL of water and 20 mL of acetonitrile, and concentrated hydrochloric acid was added dropwise to adjust the pH to 1-2, yielding a crude solution of compound **5I.** MS [M+H]⁺: calcd. 398.20, found 398.0.

### (5) Preparation of compound LM-L.

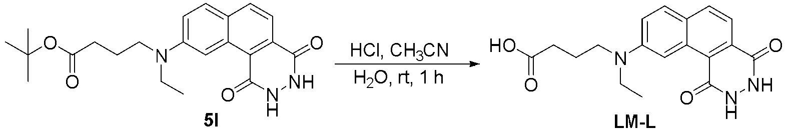

To the crude solution of compound **5I** obtained from the previous step was added dropwise 4 M HCl in dioxane (48 mL, 192.0 mmol). After complete addition, the reaction was stirred at room temperature for 1 hour. The reaction was monitored by LC-MS and stopped once most of compound **5I** had been converted. Most of the organic solvents were removed by rotary evaporation under reduced pressure using a water pump. A small amount of DMF was added to the resulting crude mixture until it became clear. The crude solution was directly injected as a liquid sample and purified by reverse-phase C18 column chromatography (CH₃CN/H₂O with 0.1% formic acid) to afford pure compound **LM-L** (530 mg, 1.6 mmol, 84% yield). ¹H NMR (400 MHz, DMSO) δ 11.73 (s, 3H), 9.33 (s, 1H), 8.11 (d, *J* = 8.6 Hz, 1H), 7.90 (d, *J* = 9.1 Hz, 1H), 7.59 (d, *J* = 8.5 Hz, 1H), 7.39 (dd, *J* = 9.1, 2.7 Hz, 1H), 3.52 (q, *J* = 7.1 Hz, 2H), 3.45 (t, *J* = 7.6 Hz, 2H), 2.35 (t, *J* = 7.3 Hz, 2H), 1.85 (p, *J* = 7.4 Hz, 2H), 1.19 (t, *J* = 6.9 Hz, 3H). MS [M+H]⁺: calcd. 342.14, found 342.0.

### Carboxyl activation of LM-L.

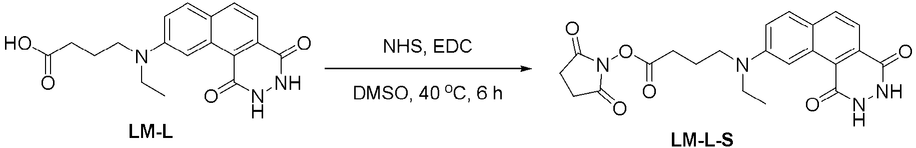

Compound **LM-L** (200 mg, 0.59 mmol) and NHS (276 mg, 2.36 mmol) were charged into a Schlenk tube, followed by the addition of DMSO (10 mL). After purging with argon, the mixture was stirred at 40°C for 5 minutes. Subsequently, the entire portion of EDC (344 mg, 1.77 mmol) was rapidly added to the reaction mixture, and the resulting mixture was stirred at 40°C for 6 hours. The reaction was monitored by LC-MS and stopped when the conversion of the target product exceeded 80%. Upon completion, the DMSO solution of activated ester **(LM-L-S)** was obtained, which could be directly used for labeling. MS [M+H]⁺: calcd. 439.15, found 438.9.

### Example 13

The synthetic route of **LM-M** is shown as follows:

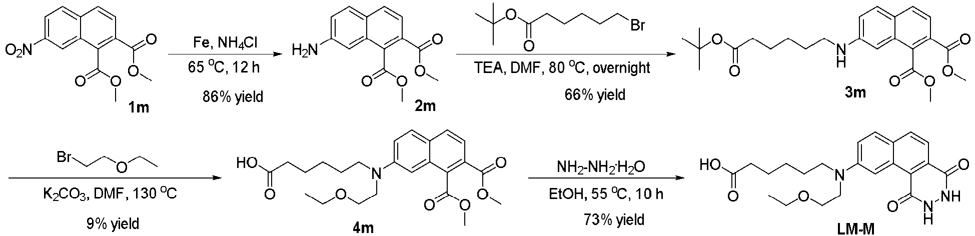

### (1) Preparation of compound 2m.

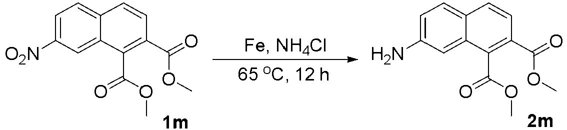

Compound **1m** (3 g, 10.4 mmol) was dissolved in methanol (90 mL), after which reduced iron powder (5.8 g, 104 mmol) was added to form a mixture. Ammonium chloride (2.8 g, 52 mmol) was dissolved in H₂O (30 mL) and then added to the above mixture. The reaction was stirred at 65 °C for 12 hours, and the process was monitored by TLC until most of the starting material had been converted. After the reaction, the mixture was filtered through Celite, and the filtrate was collected. Methanol was removed by vacuum rotary evaporation, followed by extraction with dichloromethane. The organic phase was collected and dried over anhydrous sodium sulfate; the crude compound **2m** (2.3 g, 8.9 mmol, 86% yield) was then obtained after the solvent was removed by rotary evaporation. MS [M+H]⁺: calcd. 259.08, found 228.4.

### (2) Preparation of compound 3m.

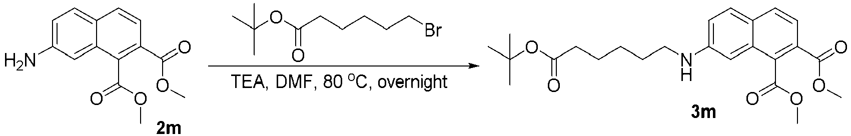

Compound **2m** (1 g, 3.9 mmol) was dissolved in DMF (15 mL), after which tert-butyl 6-bromohexanoate (6.4 mL, 31.2 mmol) and triethylamine (4.3 mL, 31.2 mmol) were added. The mixture was heated at 80 °C for 12 hours. The reaction was monitored by TLC (DCM, Rf = 0.7) until most of the starting material was completely converted. DMF (15 mL) was added to clarify the solution, and this clear solution was directly subjected to reverse-phase purification using a C18 column with eluent (CH₃CN/H₂O containing 0.1% TFA), affording pure compound **3m** (1.1 g, 2.5 mmol, 66% yield). Two batches were prepared in parallel.

### (3) Preparation of compound 4m.

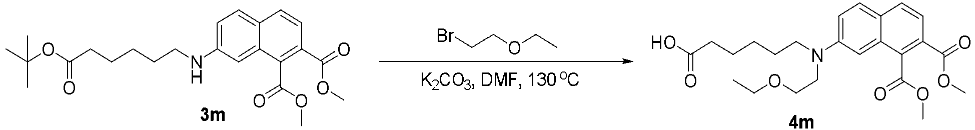

Compound **3m** (1.87 g, 4.4 mmol), DMF (30 mL), 1-bromoethoxyethane (5.2 mL, 44 mmol), and potassium carbonate (1.82 g, 13.2 mmol) were sequentially added to a sealed tube. The reaction was heated at 130 °C for 12 hours and monitored by TLC (DCM : MeOH = 10:1, Rf = 0.5) until complete conversion of the starting material. After completion, the mixture was cooled to room temperature and filtered. The resulting filtrate was directly subjected to reverse-phase purification using a C18 column (CH₃CN/H₂O with 0.1% TFA) to afford crude compound **4m.** Further purification by silica gel column chromatography (DCM/MeOH) gave pure compound **4m** (180 mg, 0.4 mmol, 9% yield). MS [M+H]⁺: calcd. 446.21, found 446.0.

### (4) Preparation of compound LM-M.

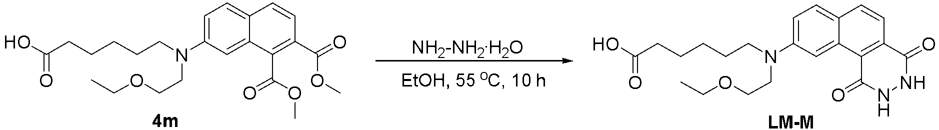

Compound **4m** (180 mg, 0.4 mmol) was dissolved in ethanol (8 mL), and hydrazine hydrate (405 µL, 8 mmol) was added. The mixture was heated and stirred at 50 °C for 10 hours. After the reaction was completed, the solvent and excess hydrazine hydrate were removed by vacuum rotary evaporation. 1 N hydrochloric acid was added dropwise until the solution became weakly acidic, and DMF (5 mL) was added to dissolve the sample, resulting in a clear solution. This clear solution was used directly for reverse-phase purification. Purification via a reverse-phase C18 column (CH₃CN/H₂O with 0.1% TFA) afforded pure compound **LM-M** (120 mg, 0.29 mmol, 73% yield). ¹H NMR (400 MHz, DMSO) δ 11.73 (s, 2H), 9.35 (s, 1H), 8.11 (d, *J* = 8.6 Hz, 1H), 7.89 (d, *J* = 9.2 Hz, 1H), 7.59 (s, 1H), 7.36 (dd, *J* = 9.2, 2.7 Hz, 1H), 3.62 (dt, *J* = 9.5, 5.0 Hz, 4H), 3.48 (q, *J* = 6.9 Hz, 4H), 2.23 (t, *J* = 7.3 Hz, 2H), 1.60 (dq, *J* = 30.7, 7.4 Hz, 4H), 1.43 - 1.34 (m, 2H), 1.11 (t, *J* = 7.0 Hz, 3H). MS [M+H]⁺: calcd. 414.20, found 414.0.

**The carboxyl activation procedure was the same as that for LM-L.**

### Example 14

The synthetic route of **LM-N** is shown as follows:

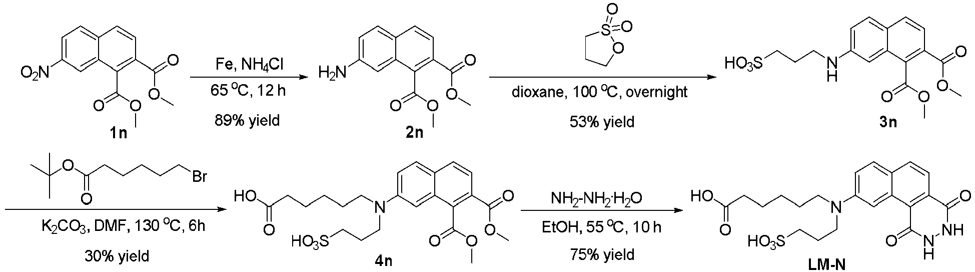

### (1) Preparation of compound 2n.

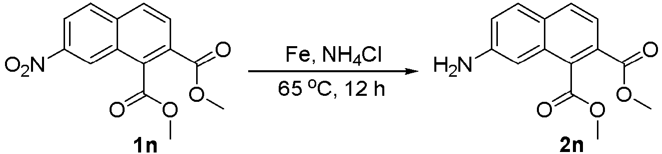

Compound **1n** (3 g, 10.4 mmol) was dissolved in methanol (90 mL), after which reduced iron powder (5.8 g, 104 mmol) was added and stirred to form a mixture. Ammonium chloride (2.8 g, 52 mmol) was dissolved in H₂O (30 mL) and then added to the above mixture. The reaction solution was heated under reflux at 65 °C for 12 hours, and the reaction was monitored by TLC (DCM, RF= 0.3) until completion. After the reaction, the mixture was filtered through Celite, and the filtrate was collected. Methanol was removed by vacuum rotary evaporation, followed by extraction with dichloromethane. The organic phase was dried over anhydrous sodium sulfate, crude compound **2n** (2.4 g, 9.3 mmol, 89% yield) was then obtained after removing solvent. MS [M+H]⁺: calcd. 259.08, found 228.4.

### (2) Preparation of compound 3n.

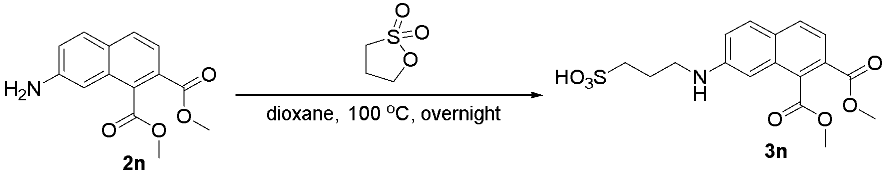

Compound **2n** (1 g, 3.9 mmol) was dissolved in dioxane (20 mL), after which propanesultone (2.4 g, 19.3 mmol) was added. The mixture was heated at 100 °C for 12 hours under argon protection. The reaction was monitored by LC-MS until most of the starting material was converted. After completion, the mixture was cooled to room temperature, and DCM (40 mL) was added to precipitate the product. The precipitate was collected by filtration and then dissolved in water (20 mL), after which this clear solution was directly used for reverse-phase purification. Purification by reverse-phase C18 column chromatography (CH₃CN/H₂O with 0.1% TFA) afforded pure compound **3n** (780 mg, 2.1 mmol, 53% yield). MS [M+H]⁺: calcd. 382.09, found 382.0.

### (3) Preparation of compound 4n.

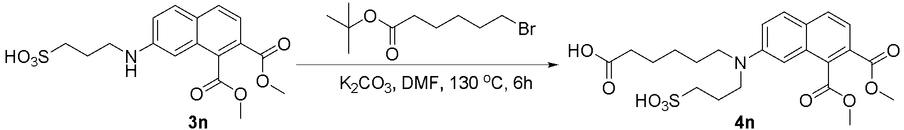

Compound **3n** (500 mg, 1.3 mmol), DMF (15 mL), tert-butyl 6-bromohexanoate (5.5 mL, 26.2 mmol), and potassium carbonate (542 mg, 3.9 mmol) were sequentially added to a sealed tube. The reaction was heated at 130 °C for 6 hours and monitored by LC-MS until most of the starting material was converted. After completion, the mixture was cooled to room temperature, and the filtrate collected by filtration was directly subjected to reverse-phase purification using a C18 column (CH₃CN/H₂O with 0.1% TFA) to afford crude compound **4n.** Further purification by silica gel column chromatography gave pure compound **4n** (200 mg, 0.4 mmol, 30% yield). MS [M-H]⁻: calcd. 494.16, found 494.0.

### (4) Preparation of compound LM-N.

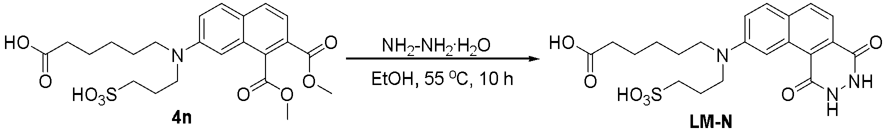

Compound **4n** (200 mg, 0.4 mmol) was dissolved in ethanol (8 mL), and hydrazine hydrate (405 µL, 8 mmol) was added. The mixture was heated and stirred at 55 °C for 10 hours. After the reaction was completed, the solvent and excess hydrazine hydrate were removed by vacuum rotary evaporation. 1 N hydrochloric acid was added dropwise until the solution became weakly acidic, and water (5 mL) was added to dilute the sample, resulting in a clear solution. This clear solution was directly used for reverse-phase purification using a C18 column (CH₃CN/H₂O with 0.1% TFA), affording pure compound **LM-N** (140 mg, 0.3 mmol, 75% yield). ¹H NMR (400 MHz, DMSO) δ 9.68 (s, 1H), 8.25 (d, *J* = 8.6 Hz, 1H), 8.09 (s, 1H), 7.80 (s, 1H), 7.68 (s, 1H), 3.65 (t, *J* = 8.1 Hz, 2H), 3.53 (t, *J* = 8.1 Hz, 2H), 2.56 (t, *J* = 7.3 Hz, 2H), 2.18 (t, *J =* 7.3 Hz, 2H), 1.91-1.81 (m, 2H), 1.57-1.47 (m, 4H), 1.34 (q, *J =* 7.4 Hz, 2H). MS [M-H]⁻: calcd. 462.14, found 461.9.

**The carboxyl activation procedure was the same as that for LM-L.**

### Example 15

The synthetic route of **LM-O** is shown as follows:

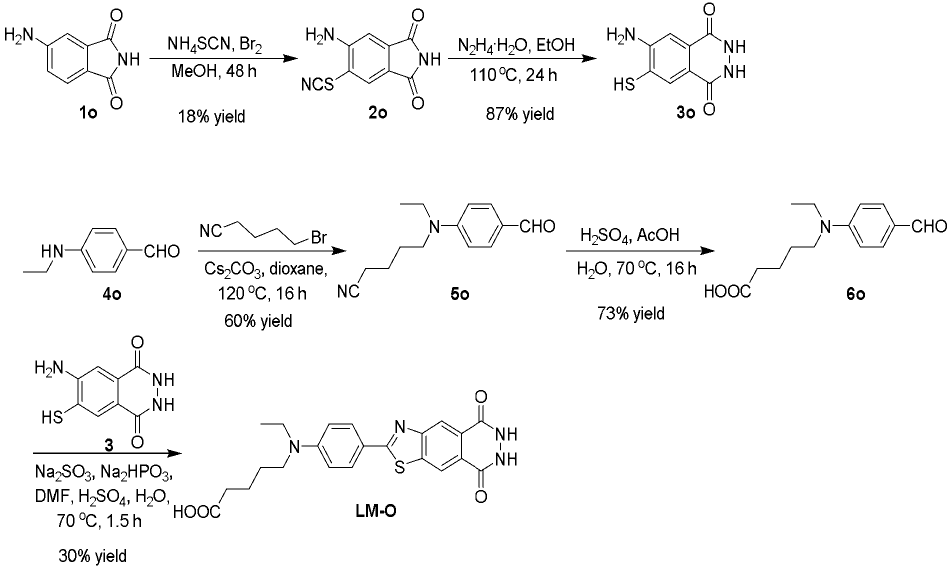

### (1) Preparation of compound 2o.

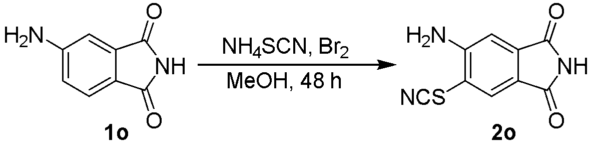

Compound **1o** (3.2 g, 19.7 mmol) and NH₄SCN (3 g, 39.5 mmol) were added to methanol (150 mL). The mixture was stirred in an ice bath and cooled to 0 °C. Bromine (1.7 mL) was diluted with methanol (50 mL) and then added dropwise to the ice-bath mixture via a constantpressure dropping funnel. After completion of the dropwise addition, the reaction was carried out in an ice-water bath for 1 hour, followed by reaction at 25 °C for 48 hours. The reaction was monitored by LC-MS until most of the starting material was consumed. Pure compound **2o** (777 mg, 18% yield) was obtained by purification using a reverse-phase C18 column (CH₃CN/H₂O with 0.1% TFA). MS [M+H]⁺: calcd. 220.0, found 220.4. This step was repeated multiple times for enough compound **2o**.

### (2) Preparation of compound 3o.

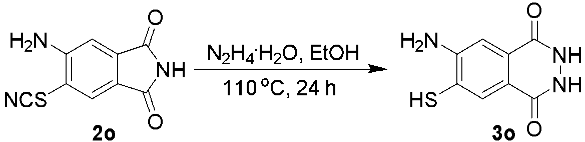

Compound **2o** (1.5 g, 6.85 mmol) and hydrazine hydrate (20 g, 400.0 mmol) were added to ethanol (20 mL). The mixture was heated under reflux for 24 hours, and the reaction was monitored by LC-MS until completion. After the reaction, ethanol and hydrazine hydrate were removed by vacuum rotary evaporation. The pH was then adjusted to 2-3 with 1 N HCl, yielding crude compound **3o**. Pure compound **3o** (1.24 g, 87% yield) was obtained by purification using a reverse-phase C18 column (CH₃CN/H₂O with 0.1% TFA). MS [M+H]⁺: calcd. 210.0, found 210.1.

### (3) Preparation of compound 5o.

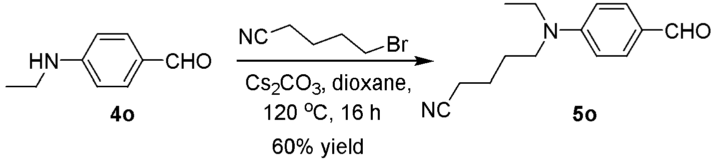

Compound **4o** (1.0 g, 6.71 mmol), cesium carbonate (4.3 g, 13.2 mmol), and pentabromovaleronitrile (22 g, 135.8 mmol) were added to 1,4-dioxane (2 mL). Under argon protection the mixture was heated under reflux for 16 hours, and the reaction was monitored by TLC until completion. Upon completion, the solvent was removed by rotary evaporation to give a crude mixture. Further purification by silica gel column chromatography (hexane/EA = 10:1) afforded pure compound **5o** (927 mg, 60% yield). MS [M+H]⁺: calcd. 231.1, found 231.5. This step was repeated multiple times for enough compound **5o**.

### (4) Preparation of compound 6o.

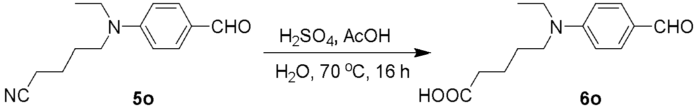

Compound **5o** (1.0 g, 4.34 mmol) was added to a mixture of concentrated sulfuric acid (1 mL), purified water (1 mL), and glacial acetic acid (1 mL). The mixture was heated at 70 °C for 16 hours, and the reaction was monitored by LC-MS until completion. After the reaction, a small amount of water was added for dilution, followed by slow portionwise addition of sodium bicarbonate to adjust the pH to 2. Purification using a C18 reverse-phase column (CH₃CN/H₂O with 0.1% FA) afforded compound **6o** (789 mg, 73% yield). MS [M+H]⁺: calcd. 250.1, found 250.5.

### (5) Preparation of compound LM-O.

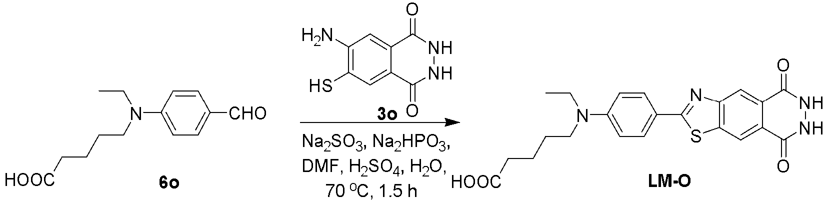

Compound **6o** (500 mg, 2.0 mmol), compound **3o** (836 mg, 4.0 mmol), sodium sulfite (160 mg, 1.27 mmol), and Na₂HPO₃ (493 mg, 3.47 mmol) were added to DMF (40 mL), followed by the addition of 1.5 M sulfuric acid (40 mL). The mixture was heated at 80 °C for 1.5 hours, the process was monitored by LC-MS until completion. After the reaction, a small amount of ice-water was added, which was then frozen in an ice bath to precipitate a solid. The crude compound **LM-O** was obtained by filtration, and further recrystallized from acetonitrile to give pure compound **LM-O** (263 mg, 30% yield). ¹H NMR (400 MHz, DMSO) δ 11.70 (s, 3H), 8.76 (s, 1H), 8.40 (s, 1H), 7.94 (d, *J* = 9.1 Hz, 2H), 6.82 (d, *J* = 9.0 Hz, 2H), 3.46 (q, *J* = 7.0 Hz, 2H), 2.33 - 2.24 (m, 2H), 1.58 (t, *J* = 3.4 Hz, 4H), 1.23 (s, 2H), 1.14 (t, *J* = 6.9 Hz, 3H). MS [M+H]⁺: calcd. 439.1, found 439.2.

### Carboxyl activation of LM-O.

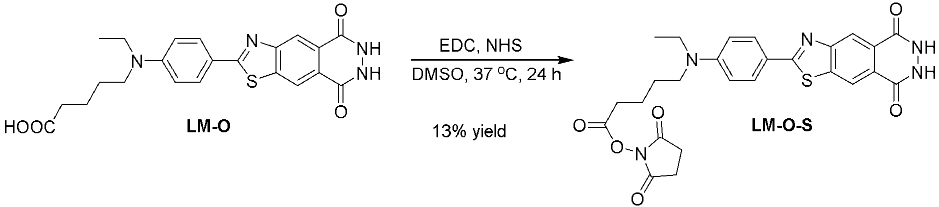

Compound **LM-O** (210 mg, 0.479 mmol) was dissolved in DMSO (10 mL), after which EDC (184 mg, 0.958 mmol) was added. The mixture was stirred at room temperature under argon protection for 20 minutes, then NHS (110 mg, 0.958 mmol) was added, and the mixture was stirred at 37 °C for 12 hours. After the reaction, compound **LM-O-S** was isolated by reverse-phase C18 column chromatography (CH₃CN/H₂O with 0.1% TFA) and lyophilized to afford pure compound **LM-O-S** (33 mg, 13% yield). MS [M+H]⁺: calcd. 536.2, found 536.1.

### Comparative example 1

This comparative example provides luminol, which is purchased from commercial sources.

### Comparative example 2

This comparative example provides ABEI (N-(4-aminobutyl)-N-ethylisoluminol), which is purchased from commercial sources. ABEI-A is an activated intermediate of ABEI, with a structural formula of 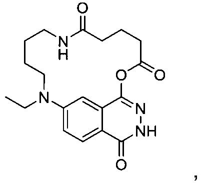 It is prepared according to the method described in the specification of Patent WO2012066003A1 and can be directly used for protein labeling.

### Experimental example

### (I) Chemiluminescence Intensity Determination of Pure Compound

1. Preparation of test solution: First, the relevant compound was dissolved in DMF to prepare a 1×10⁻³ M DMF(Dimethylformamide) solution, then it was diluted with purified water to 1×10⁻⁸ M for later use.
2. Determination of chemiluminescence (CL) intensity: 10 µL of the 1×10⁻⁸ M solution was taken, and the test was performed on the standard oxidation system of MAGLUMI^{®} X3 from Shenzhen New Industry Biomedical Engineering Co., Ltd. The chemiluminescence intensity data within 0.1-3.1 seconds was collected, and the results are shown in Table 1 below.

**Table 1**

| Luminescent compound | 0.1-3.1s CL Intensity (unit: 10,000) | Relative CL intensity (to luminol) |
|---|---|---|
| Luminol | 42.4 | 1.0 |
| ABEI-A | 199.6 | 4.7 |
| LM-A | 249.1 | 5.9 |
| LM-B | 134.1 | 3.2 |
| LM-C | 184.5 | 4.4 |
| LM-D | 165.2 | 3.9 |
| LM-E | 215.2 | 5.1 |
| LM-F | 163.7 | 3.9 |
| LM-G | 202.2 | 4.8 |
| LM-H | 210.3 | 5.0 |
| LM-I | 253.5 | 6.0 |
| LM-J | 195.8 | 4.6 |
| LM-K | 218.4 | 5.2 |
| LM-L | 114.0 | 2.7 |
| LM-M | 28.9 | 0.7 |
| LM-N | 129.8 | 3.1 |
| LM-O | 101.6 | 2.4 |

The results demonstrate that, with the exception of a few labels, most exhibit a higher CL intensity than luminol. Notably, the CL intensity of LM-A, LM-E, LM-G, LM-H, LM-I, and LM-K surpasses that of ABEI-A.

### (II) CL Intensity Dermination of Luminescent Conjugates

The conjugates were formed by coupling luminescent compounds with proteins, and the determination of their CL intensity follows the following steps:
1) Activation of carboxyl groups: All carboxyl-containing labels must undergo carboxyl group activation to convert carboxyl groups into NHS-activated esters before being labeled with BSA (bovine serum albumin). Specifically, dissolve the luminescent compound (0.05 mmol) in DMSO (1 mL), then add EDC (0.2 mmol), stir the mixture at room temperature for 20 minutes under argon protection, followed by adding NHS (0.2 mmol) and continuing to stir the mixture at 40°C for 12 hours. After the reaction is completed, monitor the reaction conversion using LC-MS and calculate the molar concentration of the NHS-activated ester based on the monitoring result; the reaction solution of activated ester is directly used for labeling.
2) BSA labeling: Weigh 1 mg of BSA (bovine serum albumin) into a vial, dissolve it in 0.1 mL of 0.1 M NaHCO₃ (sodium bicarbonate) solution to a final concentration of 10 mg/mL, corresponding to a BSA molar concentration of approximately 1.5×10⁻⁴ M. According to a labeling molar ratio of 10:1, slowly add the aforementioned NHS-activated ester solution and ABEI-A solution dropwise into the BSA solution under stirring. Stir the mixture at room temperature for 1 hour to react, and then desalt it into 0.1 mL of 0.01 M PBS (phosphate-buffered saline) solution. The final concentration of the BSA-labeled conjugate is approximately 1.5×10⁻⁴ M.
3) CL intensity determination of BSA conjugates: Dilute the BSA conjugate to a concentration of 1.5×10⁻⁸ M using 0.1 M PBS (phosphate-buffered saline) and set aside for use. Take 10 µL of the 1.5×10⁻⁸ M analyte solution to performed on the standard oxidation system of MAGLUMI^{®} X3 from Snibe Diagnostic. Collect the chemiluminescence intensity data within the time range of 0.1-3.1 seconds; the results are shown in Table 2.

**Table 2**

| BSA Conjugate | 0.1-3.1s CL intensity (unit: 10,000) | Relative CL intensity (to ABEI-BSA) |
|---|---|---|
| ABEI-BSA | 220.1 | 1.0 |
| L M-A-BSA | 310.5 | 1.4 |
| LM-B-BSA | 216.7 | 1.0 |
| LM-C-BSA | 33.0 | 0.1 |
| LM-D-BSA | 304.4 | 1.4 |
| LM-E-BSA | 224.6 | 1.0 |
| LM-F-BSA | 262.1 | 1.2 |
| LM-G-BSA | 360.8 | 1.6 |
| LM-H-BSA | 433.9 | 2.0 |
| LM-I-BSA | 121.4 | 0.6 |
| LM-J-BSA | 282.5 | 1.3 |
| LM-K-BSA | 388.2 | 1.8 |
| LM-L-BSA | 551.9 | 2.5 |
| LM-M-BSA | 724 | 3.3 |
| LM-N-BSA | 550.7 | 2.5 |
| LM-O-BSA | 314.2 | 1.4 |
| Note: molar ratio, luminescent Compound: BAS = 10:1. | | |

The results demonstrated that the detected chemiluminescence (CL) intensity of BSA conjugates labeled with LM-H, LM-L, LM-M, and LM-N was twice as high as that of ABEIlabeled BSA. For BSA labeled with LM-A, LM-D, LM-F, LM-G, LM-J, LM-K, and LM-O, their detected CL intensity was increased to varying degrees relative to ABEI. LM-B and LM-E exhibited CL intensity comparable to that of ABEI. Although the CL intensity of LM-I was lower than that of ABEI, it still met the chemiluminescence performance requirements for detection.

### (III) Coupling Ratio of Labels

Using the activated form of ABEI (ABEI-A) as a control, this experiment verified the coupling ratio between the activated form of LM-H and the labeled protein. Bovine Serum Albumin (BSA) was further used as the protein for coupling ratio verification of the label. Mass spectrometry was employed to determine how many Chemiluminescent label molecules could be conjugated to one BSA protein. Specifically, if one BSA protein was labeled with 2 label molecules, the coupling ratio was expressed as 2:1. The detection results are shown in Table 3.

**Table 3**

| BSA Conjugate | Labeling Ratio | Coupling Ratio |
|---|---|---|
| ABEI-BSA | 10:1 | 4:1 - 6:1 |
| | 20:1 | 10:1 - 12:1 |
| LM-H-BSA | 10:1 | 6:1 - 8:1 |
| | 20:1 | 14:1 - 16:1 |

The results demonstrated that the coupling ratio of activated LM-H to BSA was significantly higher than that of ABEI. This phenomenon can be attributed to the following reasons: the activation of ABEI necessitates an intermediate reaction to convert amino groups into carboxyl groups, resulting in the formation of an activated intermediate with a long chain. During protein labeling, the oscillation of this long chain gives rise to steric hindrance. In contrast, the activated structure of LM-H features a short chain, which can surmount the aforementioned steric hindrance, thereby increasing the coupling ratio and further enhancing the luminous intensity of the labeled product.

### (IV) Stability verification

To verify the stability of the aforementioned labels, the prepared label-BSA conjugate was incubated at 37°C (with a normal storage temperature of 2-8°C). Its CL intensity was continuously monitored over 7 days, and the relative deviation of the intensity change was calculated using the formula: (Initial CL Intensity - CL Intensity on Day 7) / Initial CL Intensity × 100%. The BSA-label conjugate was tested at a concentration of 1.5×10⁻⁸ M. On Days 1, 3, 5, and 7, 10 µL of the test solution was aliquoted and assayed on the SNIBE MAGLUMI^{®} X3 platform. CL intensity data were collected over the time window of 0.1-3.1 seconds, and the results are presented in Table 4 below.

**Table 4**

| BSA Conjugate | 0.1-3.1s CL intensity | | | | Deviati on% |
|---|---|---|---|---|---|
| | First day | Third day | Fifth day | Seven day | |
| ABEI-BSA | 2201130 | 2094073 | 1991017 | 1735657 | 21.1 |
| LM-A-BSA | 3105445 | 2824792 | 2644585 | 2466755 | 20.6 |
| LM-B-BSA | 2166304 | 1909104 | 1650783 | 1498835 | 30.8 |
| LM-C-BSA | 330382 | 304141 | 289357 | 231454 | 29.9 |
| LM-D-BSA | 3044627 | 2896479 | 2475616 | 2255946 | 25.9 |
| LM-E-BSA | 2246191 | 2058969 | 1814278 | 1648991 | 26.6 |
| LM-F-BSA | 2620739 | 2405762 | 2004318 | 1897785 | 27.6 |
| LM-G-BSA | 3609517 | 3435041 | 3248659 | 2872598 | 20.4 |
| LM-H-BSA | 4338443 | 4121820 | 3958659 | 3487569 | 19.6 |
| LM-I-BSA | 1213897 | 1147526 | 1087934 | 891435 | 26.6 |
| LM-J-BSA | 2825454 | 2701217 | 2545759 | 2347789 | 16.9 |
| LM-K-BSA | 3882567 | 3523595 | 3325482 | 3091133 | 20.4 |
| Note: molar ratio, Luminescent compound: BAS = 10:1_{∘} | | | | | |

s indicated in the table above, the 7-day deviation of most labels in this protocol is within 40%, which meets practical application requirements. Notably, the deviations of LM-H and LM-J are as low as 19.6% and 16.9%, respectively.

From the described description, it can be determined that the described examples of the present disclosure achieved the following technical effects:
The luminescent label provided in the present disclosure not only had a good luminescent intensity, but also can directly couple with a protein to generate a luminescent label intermediate under the action of an activator. The labeling process is simple, and the steric hindrance is lower during labeling, which can effectively improve the labeling efficiency while ensuring the luminescence performance.

## Claims

1. A luminescent label, wherein the luminescent label has a structure as represented by general formula (I): 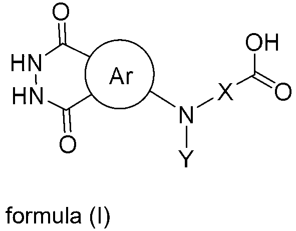
wherein Ar is any one selected from general formulas (II)-(IV): 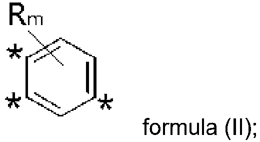 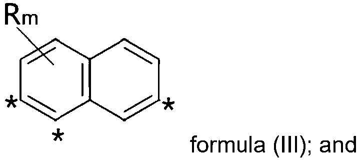 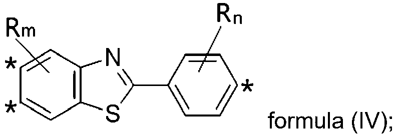
m is an integer between 0 and 2, and when m = 2, each R is the same or different;
n is an integer between 0 and 4, and when n ≥ 2, each R is the same or different;
R is selected from C1-C10 alkyl groups;
X is selected from C1-C10 alkylene groups, and C atom or H atom thereof may be optionally substituted by O atom, S atom, an aryl group or a heteroaryl group; and
Y is selected from C1-C10 alkyl groups or C1-C10 alkylsulfonic acid groups, and C atom in the alkyl groups may be optionally substituted by O atom, S atom, an aryl group or a heteroaryl group.

2. The luminescent label according to claim 1, wherein R is selected from C1-C3 alkyl groups;
and/or m is 0 or 1;
and/or n is 0;
and/or X is selected from C1-C6 alkylene groups, and wherein C atom may be optionally substituted by O atom;
and/or Y is selected from C1-C7 alkylsulfonic acid groups or C1-C6 alkyl groups, and C atom in the alkyl groups may be optionally substituted by O atom.

3. The luminescent label according to claim 1, wherein R is selected from C1-C2 alkyl groups;
and/or m is 0;
and/or X is selected from C3-C6 alkylene groups, and wherein C atom may be optionally substituted by O atom;
and/or Y is selected from C2-C4 alkylsulfonic acid groups or C1-C5 alkyl groups, and C atom in the alkyl groups may be optionally substituted by O atom.

4. The luminescent label according to claim 1, wherein the luminescent label is at least one selected from the following compounds:

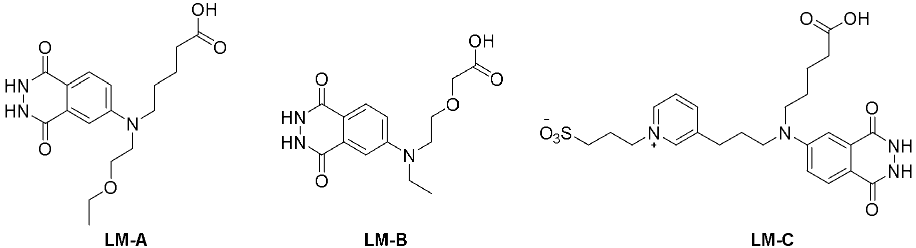

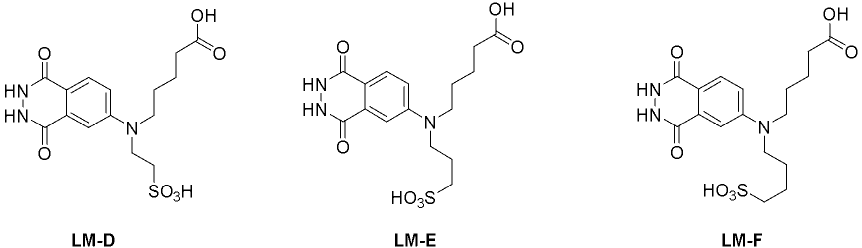

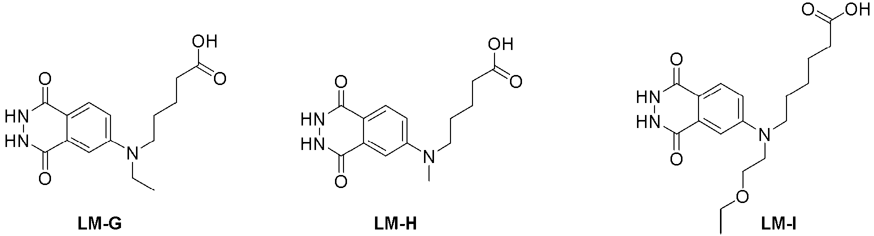

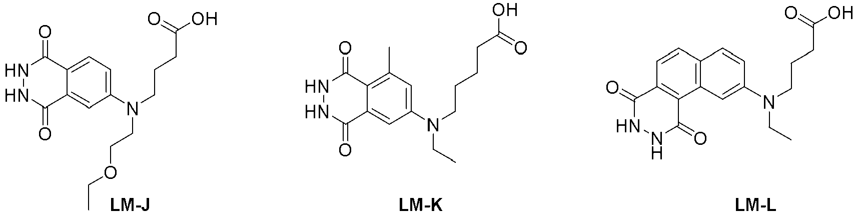

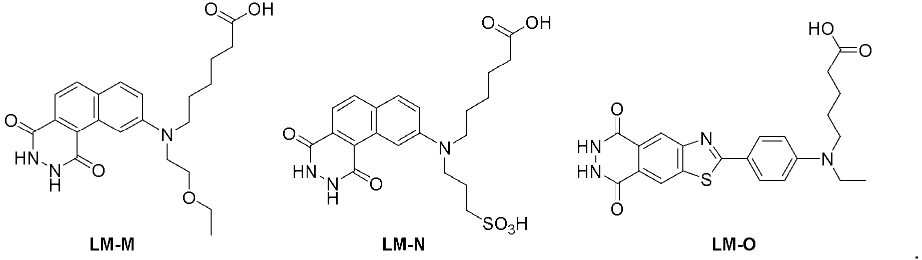

5. A luminescent label intermediate solution, wherein components of the luminescent label intermediate solution comprise a luminescent label intermediate and an organic solvent, wherein the luminescent label intermediate is formed by activating the luminescent label according to any one of claims 1 to 4 via an activator.

6. The luminescent label intermediate solution according to claim 5, wherein the activator comprises an amide condensation agent; and
preferably, the amide condensation agent comprises at least one of 1-(3-dimethylaminopropyl)-3-ethylcarbodiimide hydrochloride or dicyclohexylcarbodiimide.

7. The luminescent label intermediate solution according to claim 6, wherein the activator further comprises N-hydroxysuccinimide, and the luminescent label intermediate has a structure as represented by formula (V):

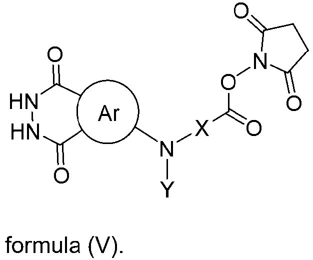

8. A preparation method for the luminescent label intermediate solution according to any one of claims 5 to 7, wherein the preparation method comprises:
dispersing the luminescent label in the organic solvent, and adding the activator and mixing same to perform an activation reaction, so that the luminescent label is activated by the activator to form a luminescent label intermediate, so as to obtain the luminescent label intermediate solution.;
the temperature of the activation reaction is 10-50°C, and the time of the activation reaction is 8-20 h.

9. The preparation method for the luminescent label intermediate solution according claim 8, the activator comprises an amide condensation agent and N-hydroxysuccinimide.

10. The preparation method for the luminescent label intermediate solution according claim 9, the molar ratio of the N-hydroxysuccinimide to the luminescent label is ≥1, and preferably (1.2-4):1.

11. Use of the luminescent label of any one of claims 1 to 4 or the luminescent label intermediate solution of any one of claims 5 to 7 in preparation of a luminescent label complex.

12. A luminescent label complex, wherein the luminescent label complex is formed by coupling the luminescent label of any one of claims 1 to 4 and a protein, and the luminescent label complex has a structure as represented by formula (VI):

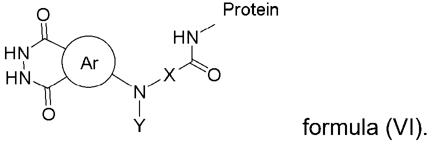

13. A preparation method for the luminescent label complex of claim 12, wherein the preparation method for the luminescent label complex comprises: mixing the luminescent label intermediate solution of any one of claims 5 to 7 with a protein to perform a coupling reaction, so as to obtain the luminescent label complex.

14. Use of the luminescent label of any one of claims 1 to 4, the luminescent label intermediate solution of any one of claims 5 to 7, or the luminescent label complex of claim 12 in luminescent immunoassay.

15. A kit, wherein the kit comprises the luminescent label of any one of claims 1 to 4, the luminescent label intermediate solution of any one of claims 5 to 7, or the luminescent label complex of claim 12.
